# EUROPEAN PATENT APPLICATION

(11) **EP 4 591 797 A1**
(43) Date of publication of application: **30.07.2025**
(21) Application number: 25153990.4
(22) Date of filing: 27.01.2025
(51) Int. Cl.: A61B 6/00, A61B 6/58, A61B 6/42

(54) **A RADIOGRAPHIC IMAGING DEVICE AND POSITIONING METHOD THEREOF**

(30) Priority: 29.01.2024 CN 202410125150
(71) Applicant: Wuhan Mindray Bio-Medical Scientific Co., Ltd., Wuhan City Hubei 430206 (CN); Shenzhen Mindray Bio-Medical Electronics Co., Ltd., Shenzhen, Guangdong 518057 (CN)
(72) Inventor: ZHANG, Zanchao, Shenzhen, 518057 (CN); LIU, Xuedong, Shenzhen, 518057 (CN); WEN, Qi, Shenzhen, 518057 (CN)
(74) Representative: KIPA AB

(57) **Abstract**

A radiographic imaging device and a positioning method thereof are provided. The radiographic imaging device includes a head, a movable detector, a first posture sensor, at least one first base station and at least one tag. The first base station is configured to transmit a signal to the tag and receive a signal returned from the tag. In the present disclosure, the movable detector is used, which can be placed without being limited to a specific location. Therefore, the detector can perform the imaging at a plurality of positions, which effectively expands the application scenario of the radiographic imaging device. Furthermore, the base station and the tag are used to position the movable detector, and the posture sensor is used to measure the posture of the detector, which facilitates the radiographic imaging device to complete the position matching of the head and the movable detector (e.g., making the two meet a preset position relationship).

## Description

### TECHNICAL FIELD

The present disclosure relates to radiographic imaging, and particularly to a radiographic imaging device and positioning method thereof.

### BACKGROUND

As a common imaging system in medical digital imaging, radiographic imaging device is widely used in physical examination and conventional medical imaging diagnosis. The radiographic imaging device is a device that uses radioactive radiation rays (such as X-radiation rays) to penetrate the object being examined to perform imaging.

Take Digital Radiography (DR) as an example, duo to its advantages such as fast imaging speed, low radiation dose, clear and delicate images and the ability to examine the whole body, it is widely used in clinical examinations and has become one of the main assistant means for doctors to diagnose diseases. Generally, a DR device has a head and a detector. The head is a component configured to transmit X-radiation rays, and the detector is a component configured to receive X-radiation rays that have passed through the object being examined. The detector converts the received X-radiation rays into electrical signals to form an image. Therefore, during imaging, the detector needs to be located in the "optical path" of the X-radiation rays transmitted by the head, and, in order to avoid distortion in the image, it is generally also required that the head and the detector are aligned.

How to achieve the desired relative position between the head and the detector is a problem to be solved.

### SUMMARY

The present disclosure provides radiographic imaging devices and positioning method thereof, which will be described below.

In one embodiment, a mobile radiographic imaging device is provided, which may include a movable body, a head, a controller and a movable detector, where
the movable body includes a body and a movable assembly, where the movable assembly brings the body to move;
the head is configured to generate radiation rays;
the detector is configured to receive the radiation rays for imaging;
the head is arranged with at least one base station, and the detector is arranged with a first posture sensor and at least one tag, wherein, the base station is configured to transmit a signal to the tag and receive a signal returned from the tag, and the first posture sensor is configured to measure a posture of the detector; and
the controller is configured to calculate a relative position between the base station and the tag according to the signals transmitted and received by the base station, where the relative position includes a relative distance and a relative angle.

In one embodiment, the at least one base station includes a first base station, and the controller is configured to calculate a relative position between the first base station and the tag according to signals transmitted and received by the first base station.

In one embodiment, the first base station includes at least two antennas for transmitting and receiving signals, wherein the at least two antennas includes a first antenna and a second antenna; and
the controller is configured to calculate a first relative distance between the first antenna and the tag according to signals transmitted and received by the first antenna, calculate a first signal phase difference according to the signals transmitted and received by the first antenna and signals transmitted and received by the second antenna, and calculate the relative position between the first base station and the tag according to the first relative distance and the first signal phase difference, where the first signal phase difference is a phase difference between the signals received by the first antenna and the signals received by the second antenna.

In one embodiment, the first base station includes three antennas for transmitting and receiving signals, where the three antennas includes the first antenna, the second antenna, and a third antenna; and
the controller is configured to calculate a second signal phase difference according to the signals transmitted and received by the first antenna and signals transmitted and received by the third antenna, and calculate the relative position between the base station and the tag according to the first relative distance, the first signal phase difference and the second signal phase difference, where the second signal phase difference is a phase difference between the signals received by the first antenna and the signals received by the third antenna.

In one embodiment, the three antennas are arranged at three sites of the first base station, and the three sites are located at three vertices of a right triangle.

In one embodiment, the detector is arranged with one tag, two tags or at least three tags.

In one embodiment, three of the tags are arranged at three sites of the detector, and the three sites are not on one straight line.

In one embodiment, the detector is a flat panel detector, and the three sites are located at three corners of the flat panel detector.

In one embodiment, the controller is further configured to calculate a spatial position of the movable detector according to the relative position between one of the first base station and at least one of the tags.

In one embodiment, the controller is further configured to calculate a posture of the movable detector according to the relative position between one of the first base station and at least three of the tags.

In one embodiment, the radiographic imaging device further includes a driving assembly and a support assembly, where, the support assembly includes a first support element, and the first support element is arranged on the movable body and is connected to the head to support the head; the driving assembly drives the head to move through the first support element; and the controller is configured to control the driving assembly to drive the head to move according to the relative position and the posture of the detector to make the head to meet a preset position relationship with the detector;
or,
the radiographic imaging device further includes a support assembly, where, the support assembly include a first support element, the first support element is arranged on the movable body and is connected to the head to support the head; and the controller is configured to prompt an user according to the relative position and the posture of the detector such that the user can control the head to move to make the head to meet a preset position relationship with the detector.

In one embodiment, the preset position relationship between the head and the detector includes: a distance between the head and an imaging surface of the detector satisfying a first distance requirement, and/or a distance between the head and a first straight line satisfying a second distance requirement, where the first straight line is a straight line that is vertical to and passes through a center of the imaging surface of the detector; and the controller is configured to control the driving assembly to drive the head to move according to the relative position, which includes:
the controller in configured to calculate a spatial position of the detector according to the relative position; and
the controller is configured to acquire a spatial position of the head and, according to the spatial position of the head and the spatial position of the detector, control the driving assembly to drive the head to move to make the head and the detector to meet the distance requirement.

In one embodiment, the preset position relationship between the head and the detector comprises: an angle between an irradiation surface of the head and an imaging surface of the detector satisfying an angle requirement;
the controller is configured to control the driving assembly to drive the head to rotate according to at least the posture of the detector, which includes: the controller is configured to obtain a posture of the head, and control the driving assembly to drive the head to rotate according to the posture of the head and the posture of the detector to make the head and the detector to meet the angle requirement.

In one embodiment, the controller is further configured to calibrate the posture of the detector according to the relative position.

In one embodiment, the controller being further configured to calibrate the posture of the detector according to the relative position includes:
the controller is configured to calculate the posture of the detector according to the relative positions between the first base station and three tags; and
the controller is configured to calibrate the posture measure by the first posture sensor according to the posture calculated by the relative positions.

In one embodiment, the at least one base station further includes a second base station;
the controller is configured to calculate a relative position between the second base station and the tag according to signals transmitted and received by the second base station, where the relative position includes a relative distance and a relative angle; and
the controller is configured to calibrate the spatial position of the detector and/or the posture measure by the first posture sensor according to the relative position between the second base station and the tag.

In one embodiment, the second base station is the same as the first base station.

In one embodiment, the head is arranged with a second posture sensor configured to measure a posture of the head.

In one embodiment, the base station is a UWB-type base station, and the tag is a UWB-type tag.

In one embodiment, a mobile radiographic imaging device is provided, which may include a movable body, a head, a controller and a movable detector; where
the movable body includes a body and a movable assembly, where the movable assembly brings the body to move;
the head is configured to generate radiation rays;
the detector is configured to receive the radiation rays for imaging;
the head is arranged with at least one base station, and the detector is arranged with a first posture sensor and at least one tag, wherein, the base station is configured to transmit signals to the tag and receive signals returned from the tag, and the first posture sensor is configured to measure a posture of the detector; and
the controller is configured to calculate a relative position between the base station and the tag according to the signals transmitted and received by the base station.

In one embodiment, the at least one base station includes a first base station, and the controller is configured to calculate a relative position between the first base station and the tag according to signals transmitted and received by the first base station.

In one embodiment, the relative position includes a relative distance and a relative angle.

In one embodiment, the controller is further configured to calculate a spatial position of the movable detector according to the relative position between one of the first base station and at least one of the tags.

In one embodiment, a radiographic imaging device is provided, which may include a head, a controller, a movable detector, a first posture sensor, at least one base station and at least one tag; where:
the head is configured to generate radiation rays;
the detector is configured to receive the radiation rays for imaging;
the at least one base stations includes at least one first base station that is configured to transmit signals to the tag and receive signals returned from the tag, and the controller is configured to calculate a relative position between the first base station and the tag according to the signals transmitted and received by one of the first base station, where, the relative position includes a relative distance and a relative angle, and the first base station and the tag are such arranged that the relative position between the first base station and the tag at least represents a spatial position of the detector;
the first posture sensor is arranged at the detector and configured to measure a posture of the detector.

In one embodiment, the first base station includes at least two antennas for transmitting and receiving signals, wherein the at least two antennas includes a first antenna and a second antenna; and
the controller is configured to calculate a first relative distance between the first antenna and the tag according to signals transmitted and received by the first antenna, calculate a first signal phase difference according to the signals transmitted and received by the first antenna and signals transmitted and received by the second antenna, and calculate the relative position between the first base station and the tag according to the first relative distance and the first signal phase difference, where the first signal phase difference is a phase difference between the signals received by the first antenna and the signals received by the second antenna.

In one embodiment, the first base station includes three antennas for transmitting and receiving signals, where the three antennas includes the first antenna, the second antenna, and a third antenna; and
the controller is configured to calculate a second signal phase difference according to the signals transmitted and received by the first antenna and signals transmitted and received by the third antenna, and calculate the relative position between the base station and the tag according to the first relative distance, the first signal phase difference and the second signal phase difference, where the second signal phase difference is a phase difference between the signals received by the first antenna and the signals received by the third antenna.

In one embodiment, the three antennas are arranged at three sites of the first base station, and the three sites are located at three vertices of a right triangle.

In one embodiment, at least one of the first base stations is arranged at a specific location at the head or in space, and at least one of the tags is arranged at the detector;
or,
at least one of the tags is arranged at a specific location at the head or in space, and at least one of the first base stations is arranged at the detector.

In one embodiment, the detector is arranged with one tag, two tags or at least three tags.

In one embodiment, three of the tags are arranged at three sites of the detector, and the three sites are not on one straight line.

In one embodiment, the detector is arranged with multiple first base stations; and preferably, the detector is arranged with three first base stations; and preferably, the three first base stations are arranged at three sites of the detector and the three sites are not on one straight line.

In one embodiment, the detector is a flat panel detector, and the three sites are located at three corners of the flat panel detector.

In one embodiment, the controller is further configured to calculate a spatial position of the movable detector according to the relative position between one of the first base stations and at least one of the tags.

In one embodiment, the radiographic imaging device further includes a driving assembly and a support assembly, where, support assembly includes a first support element and/or a second support element, and the first support element is connected to the head to support the head; the driving assembly drives the head to move through the first support element; the driving assembly further drive the second support element to move, and when the detector is placed on the second support element, the second support element bring the detector to move; and the controller is configured to control the driving assembly to drive the head and/or the detector to move according to the relative position and the posture of the detector to make the head and the detector to meet a preset position relationship;
or,
the radiographic imaging device further includes a support assembly that includes a first support element and/or a second support element, where, the first support element is connected to the head to support the head; when the detector is placed on the second support element, the second support element bring the detector to move; and the controller is configured to prompt an user according to the relative position and the posture of the detector such that the user can control the head and/or the detector to move to make the head and the detector to meet a preset position relationship.

In one embodiment, the preset position relationship between the head and the detector includes: a distance between the head and an imaging surface of the detector satisfying a first distance requirement, and/or a distance between the head and a first straight line satisfying a second distance requirement, where the first straight line is a straight line that is vertical to and passes through a center of the imaging surface of the detector; and the controller is configured to control the driving assembly to drive the head and/or the detector to move according to the relative position, which includes:
the controller in configured to calculate a spatial position of the detector according to the relative position; and
the controller is configured to acquire a spatial position of the head and, according to the spatial position of the head and the spatial position of the detector, control the driving assembly to drive the head and/ or the detector to move to make the head and the detector to meet the distance requirement.

In one embodiment, the preset position relationship between the head and the detector comprises: an angle between an irradiation surface of the head and an imaging surface of the detector satisfying an angle requirement;
the controller is configured to control the driving assembly to drive the head and/or the detector to rotate according to at least the posture of the detector, which comprises: the controller is configured to acquire a posture of the head and, according to the posture of the head and the posture of the detector, control the driving assembly to drive the head and/or the detector to rotate to make the head and the detector to meet the angle requirement.

In one embodiment, the controller is further configured to calibrate the posture of the detector according to the relative position.

In one embodiment, the controller being further configured to calibrate the posture of the detector according to the relative position comprises:
the controller is configured to calculate the posture of the detector according to the relative positions between the first base station and three tags; and
the controller is configured to calibrate the posture measure by the first posture sensor according to the posture calculated by the relative positions.
the controller is configured to calibrate the posture measure by the first posture sensor according to the posture calculated by the relative positions.

In one embodiment, the head is arranged with one or more tags, and preferably, the head is arranged with at least three tags; and the controller is configured to calculate a spatial position and/or a posture of the head according to the relative position between the first base station and the tag arranged at the head;
or, the head is arranged with a second posture sensor configured to measure a posture of the head.

In one embodiment, the at least one base station further includes a second base station, the second base station is configured to transmit signals to the tag and receive signals returned from the tag, and the controller is configured to calculate a relative position between the second base station and the tag according to the signals transmitted and received by the second base station, where the relative position includes a relative distance and a relative angle;
the controller is further configured to calibrate at least one of a spatial position of the detector, a posture of the detector, a spatial position of the head and a posture of the head according to the relative position between the second base station and the tag.

In one embodiment, the second base station is arranged at the head or a specific location in space.

In one embodiment, the second base station is the same as the first base station.

In one embodiment, the first base station is a UWB-type base station and the tag is a UWB-type tag.

In one embodiment, a radiographic imaging device is provided, which may include a head, a controller, a movable detector and a first posture sensor; where
the head is configured to generate radiation rays;
the detector is configured to receive the radiation rays for imaging;
the head is arranged with at least one base station, and the detector is arranged with at least one tag, wherein, the base station is configured to transmit a signal to the tag and receive a signal returned from the tag, and the controller is configured to calculate a relative position between the base station and the tag according to the signals transmitted and received by the base station; and
the first posture sensor is arranged at the detector and configured to measure a posture of the detector.

In one embodiment, the at least one base station includes a first base station, and the controller is configured to calculate a relative position between the first base station and the tag according to signals transmitted and received by the first base station.

In one embodiment, the relative position includes a relative distance and a relative angle.

In one embodiment, the controller is configured to calculate a spatial position of the movable detector according to the relative position between one of the first base station and at least one of the tags.

In one embodiment, the radiographic imaging device further includes a driving assembly and a support assembly, where, support assembly includes a first support element and/or a second support element, and the first support element is connected to the head to support the head; the driving assembly drives the head to move through the first support element; the driving assembly further drive the second support element to move, and when the detector is placed on the second support element, the second support element bring the detector to move; and the controller is configured to control the driving assembly to drive the head and/or the detector to move according to the relative position and the posture of the detector to make the head and the detector to meet a preset position relationship;
or,
the radiographic imaging device further includes a support assembly that includes a first support element and/or a second support element, where, the first support element is connected to the head to support the head; when the detector is placed on the second support element, the second support element bring the detector to move; and the controller is configured to prompt an user according to the relative position and the posture of the detector such that the user can control the head and/or the detector to move to make the head and the detector to meet a preset position relationship.

In one embodiment, a radiographic imaging device is provided, which may include a head, a controller, a movable detector, a movable assistant device, a first posture sensor, at least one base station and at least one tag; where
the head is configured to generate radiation rays;
the detector is configured to receive the radiation rays for imaging;
the assistant device is used to place the detector during the detector receiving the radiation rays, wherein the first posture sensor is arranged at the assistant device and configured to measure a posture of the assistant device;
the at least one base station comprises at least one first base station, wherein the first base station is configured to transmit signals to the tag and receive signals returned from the tag; and
the controller is configured to calculate a relative position between the first base station and the tag according to the signals transmitted and received by one of the first base station, wherein, the first base station and the tag are such arranged that the relative position between the first base station and the tag at least represents a spatial position of the detector.

In one embodiment, the relative position includes a relative distance and a relative angle.

In one embodiment, the first base station includes at least two antennas for transmitting and receiving signals, wherein the at least two antennas includes a first antenna and a second antenna; and
the controller is configured to calculate a first relative distance between the first antenna and the tag according to signals transmitted and received by the first antenna, calculate a first signal phase difference according to the signals transmitted and received by the first antenna and signals transmitted and received by the second antenna, and calculate the relative position between the first base station and the tag according to the first relative distance and the first signal phase difference, where the first signal phase difference is a phase difference between the signals received by the first antenna and the signals received by the second antenna.

In one embodiment, the first base station includes three antennas for transmitting and receiving signals, where the three antennas includes the first antenna, the second antenna, and a third antenna; and
the controller is configured to calculate a second signal phase difference according to the signals transmitted and received by the first antenna and signals transmitted and received by the third antenna, and calculate the relative position between the base station and the tag according to the first relative distance, the first signal phase difference and the second signal phase difference, where the second signal phase difference is a phase difference between the signals received by the first antenna and the signals received by the third antenna.

In one embodiment, the three antennas are arranged at three sites of the first base station, and the three sites are located at three vertices of a right triangle.

In one embodiment, at least one of the first base stations is arranged at a specific location at the head or in space, and at least one of the tags is arranged at the detector;
or,
at least one of the tags is arranged at a specific location at the head or in space, and at least one of the first base stations is arranged at the detector.

In one embodiment, the assistant device is arranged with one tag, two tags or at least three tags.

In one embodiment, the three tags are arranged at three sites of the assistant device, and the three sites are not on one straight line.

In one embodiment, the assistant device is arranged with multiple first base stations; and preferably, the assistant device is arranged with three first base stations; and preferably, the three first base stations are arranged at three sites of the assistant device, and the three sites are not on one straight line.

In one embodiment, the three sites are three corners of the assistant device.

In one embodiment, the controller is further configured to calculate a spatial position of the movable assistant device according to a relative position between one of the first base stations and at least one of the tags.

In one embodiment, the radiographic imaging device further comprises a driving assembly, wherein, the driving assembly comprises a first support element and/or a second support element, and the first support element is connected to the head to support the head; the driving assembly drives the head to move through the first support element; and the driving assembly drives the second support element to move, and when the assistant device is placed on the second support element, the second support element bring the assistant device to move;
or,
the radiographic imaging device further comprises a support assembly that comprises a first support element and/or a second support element, wherein, the first support element is connected to the head to support the head; when the assistant device is placed on the second support element, the second support element bring the assistant device to move; and the controller is configured to prompt an user according to the relative position and the posture of the assistant device such that the user can control the head and/or the detector to move to make the head and the detector to meet a preset position relationship.

In one embodiment, the preset position relationship between the head and the detector placed in the assistant device comprises: a distance between the head and an imaging surface of the detector placed in the assistant device satisfying a first distance requirement, and/or a distance between the head and a first straight line satisfying a second distance requirement, wherein the first straight line is a straight line that is vertical to and passes through a center of the imaging surface of the detector placed in the assistant device; and the controller is configured to control the driving assembly to drive the head and/or the assistant device to move according to the relative position, which comprises:
the controller in configured to calculate a spatial position of the assistant device according to the relative position; and
the controller is configured to acquire a spatial position of the head and, according to the spatial position of the head and the spatial position of the assistant device, control the driving assembly to drive the head and/ or the assistant device to move to make the head and the detector placed in the assistant device to meet the distance requirement.

In one embodiment, the preset position relationship between the head and the detector placed in the assistant device comprises: an angle between an irradiation surface of the head and an imaging surface of the detector placed in the assistant device satisfying an angle requirement;
the controller is configured to control the driving assembly to drive the head and/or the assistant device to rotate according to at least the posture of the assistant device, which comprises: the controller is configured to acquire a posture of the head and, according to the posture of the head and the posture of the assistant device, control the driving assembly to drive the head and/or the assistant device to rotate to make the head and the detector placed in the assistant device to meet the angle requirement.

In one embodiment, the controller is further configured to calibrate the posture of the assistant device according to the relative position.

In one embodiment, the controller being further configured to calibrate the posture of the detector according to the relative position comprises:
the controller is configured to calculate the posture of the assistant device according to the relative positions between the first base station and three tags; and
the controller is configured to calibrate the posture measure by the first posture sensor according to the posture calculated by the relative positions.

In one embodiment, the head is arranged with one or more tags, and preferably, the head is arranged with at least three tags; and the controller is configured to calculate a spatial position and/or a posture of the head according to the relative position between the first base station and the tag arranged at the head;
or, the head is arranged with a second posture sensor configured to measure a posture of the head.

In one embodiment, the at least one base station further includes a second base station, the second base station is configured to transmit signals to the tag and receive signals returned from the tag, and the controller is configured to calculate a relative position between the second base station and the tag according to the signals transmitted and received by the second base station, where the relative position includes a relative distance and a relative angle; and
the controller is further configured to calibrate at least one of a spatial position of the assistant device, a posture of the assistant device, a spatial position of the head and a posture of the head according to the relative position between the second base station and the tag.

In one embodiment, the second base station is arranged at the head or a specific location in space.

In one embodiment, the second base station is the same as the first base station.

In one embodiment, the first base station is a UWB-type base station and the tag is a UWB-type tag.

In one embodiment, a positioning method for a radiographic imaging device is provided, where, the radiographic imaging device includes a head, a movable detector, a first posture sensor, at least one first base station and at least one tag, the first base station is configured to transmit a signal to the tag and receive a signal returned from the tag; and the method includes:
calculating a relative position between the first base station and the tag according to the signals transmitted and received by the first base station, where, the relative position includes a relative distance and a relative angle, and the relative position between the first base station and the tag at least represents a spatial position of the detector; and
obtaining a posture of the detector through the first posture sensor;
controlling the head and/or the detector to move according to the relative position and controlling the head and/or the detector to rotate according to at least the posture of the detector to make the head and the detector to meet a preset position relationship.

In one embodiment, the preset position relationship between the head and the detector includes: a distance between the head and an imaging surface of the head satisfying a first distance requirement, and/or, a distance between the head and a first straight line satisfying a second distance requirement, where the first straight line is a straight line that is vertical to and passes through a center of the imaging surface of the detector; and
controlling the head and/or the detector to move according to the relative position comprises:
calculating a spatial position of the detector according to the relative position; and
obtaining a spatial position of the head and, according to the spatial position of the head and the spatial position of the detector, controlling the head and/or the detector to move to make the head and the detector to meet the distance requirement.

In one embodiment, the preset position relationship between the head and the detector includes: the angle between an irradiation surface of the head and an imaging surface of the detector satisfying an angle requirement; and
controlling the head and/or the detector to rotate according to at least the posture of the detector comprises: obtaining a posture of the head, and controlling the head and/or the detector to rotate according to the posture of the head and the posture of the detector to make the head and the detector to meet the angle requirement.

In one embodiment, the radiographic imaging device is the radiographic imaging device of any embodiment described in the present disclosure.

In the radiographic imaging devices and the positioning methods of the embodiment above, the movable detector is used. The movable detector can be placed not only in a specific location but also in multiple locations to performing the imaging, which effectively expands the application scenarios of the radiographic imaging device. Furthermore, the base station and the tag are used to position the movable detector, and the posture sensor is used to measure the posture of the detector, which provide a basis for the radiographic imaging device to complete the position matching between the head and the movable detector (for example, making the two meet a preset position relationship).

### BRIEF DESCRIPTION OF THE DRAWINGS

FIG. 1 is a schematic diagram of a radiographic imaging device in one embodiment;
FIG. 2 is a schematic diagram of a radiographic imaging device in one embodiment;
FIG. 3 is a schematic diagram of a radiographic imaging device in one embodiment;
FIG. 4 (1) is a schematic diagram of a movable detector in one embodiment;

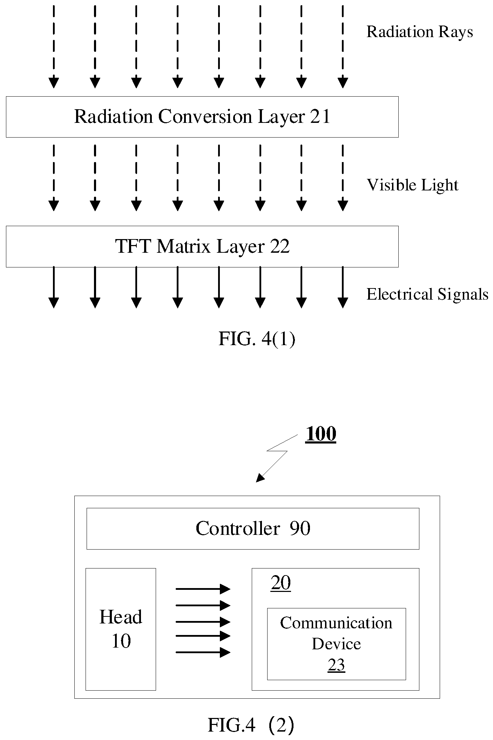

FIG. 4 (2) is a schematic diagram of a radiographic imaging device in one embodiment;

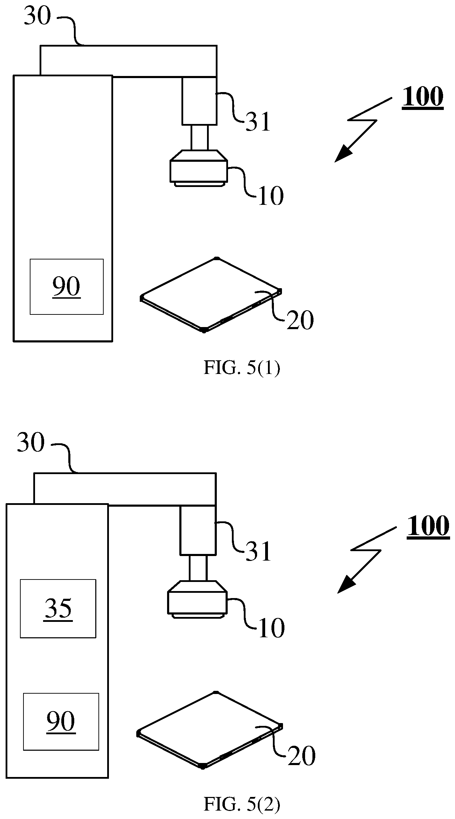

FIG. 5 (1) is a schematic diagram of a radiographic imaging device in one embodiment; and FIG. 5 (2) is a schematic diagram of a radiographic imaging device in one embodiment;

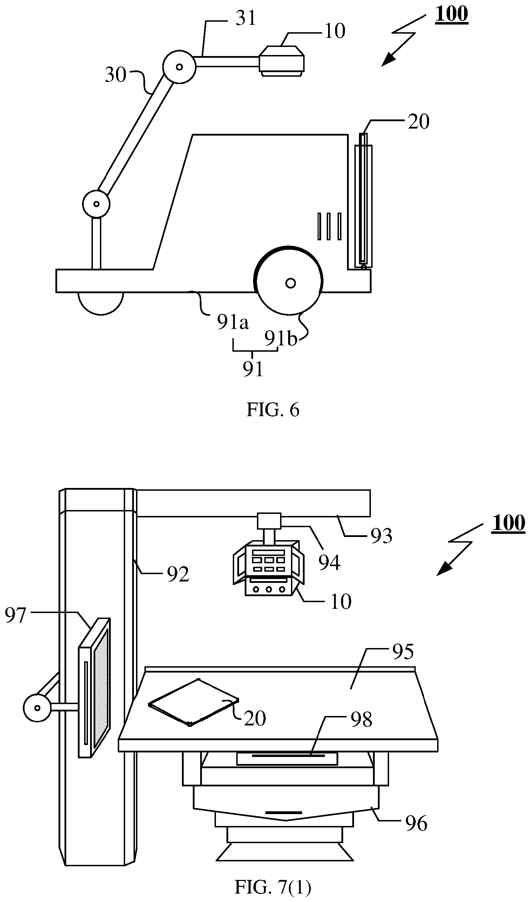

FIG. 6 is a schematic diagram of a radiographic imaging device in one embodiment;

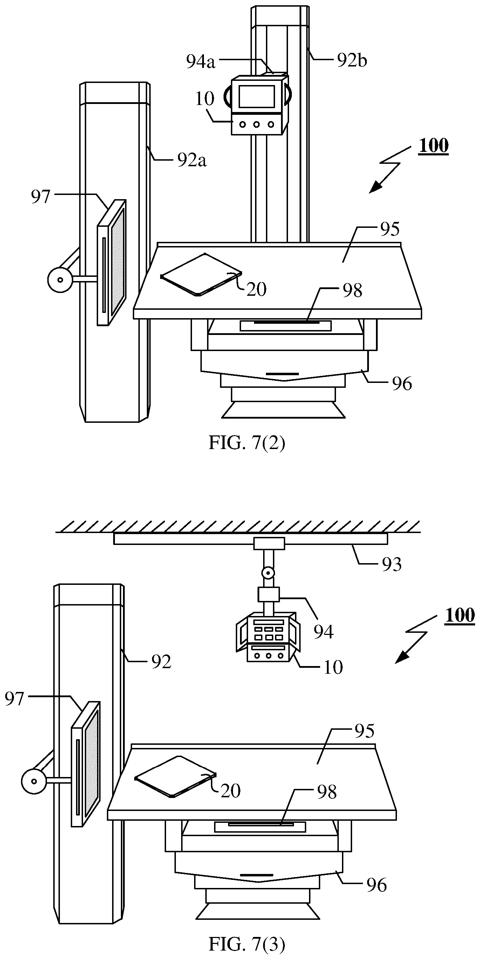

FIG. 7(1) is a schematic diagram of a radiographic imaging device in one embodiment; FIG. 7(2) is a schematic diagram of a radiographic imaging device in one embodiment; and FIG. 7(3) is a schematic diagram of a radiographic imaging device in one embodiment;

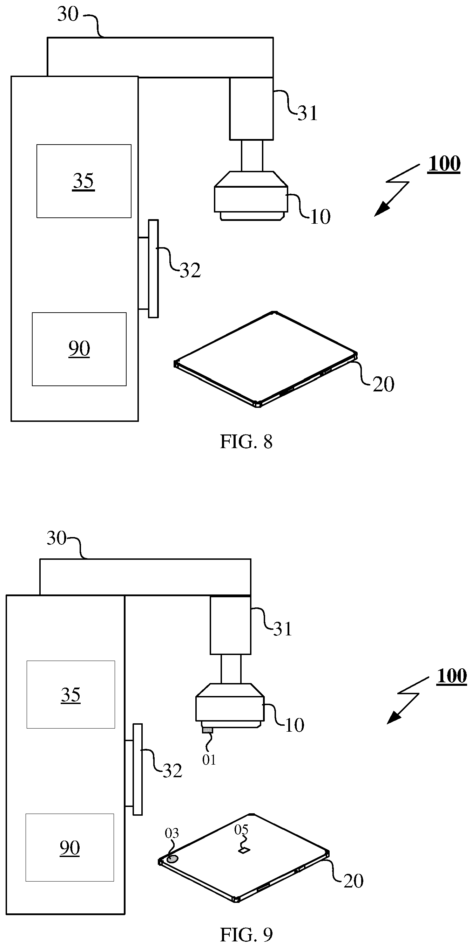

FIG. 8 is a schematic diagram of a radiographic imaging device in one embodiment;
FIG. 9 is a schematic diagram of a radiographic imaging device in one embodiment;

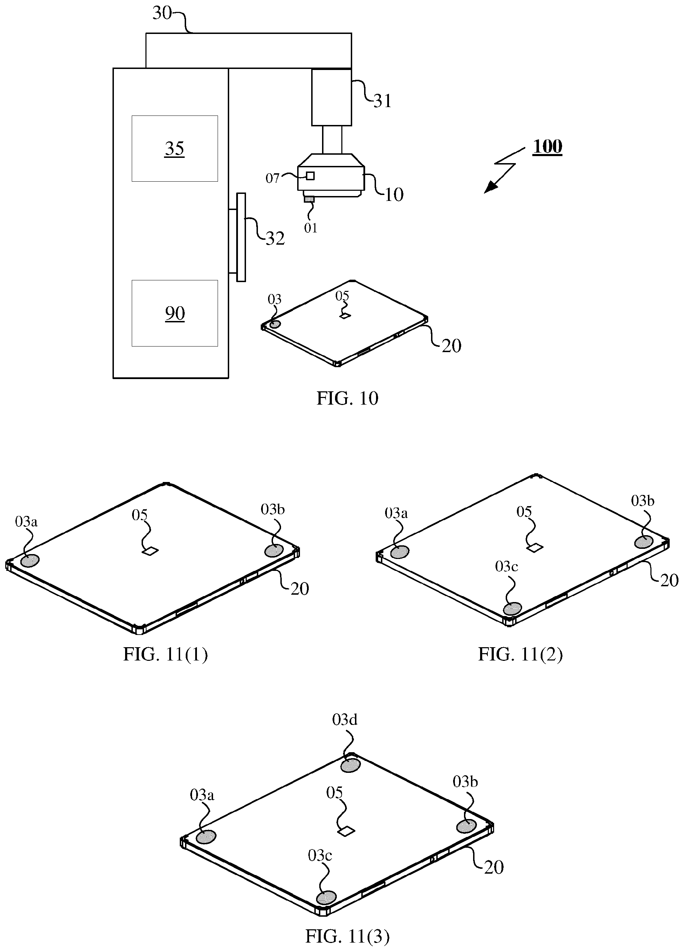

FIG. 10 is a schematic diagram of a radiographic imaging device in one embodiment;
FIG. 11 (1) is a schematic diagram of a movable detector arranged with tags in one embodiment; FIG. 11(2) is a schematic diagram of a movable detector arranged with tags in one embodiment; and FIG. 11(3) is a schematic diagram of a movable detector arranged with tags in one embodiment;

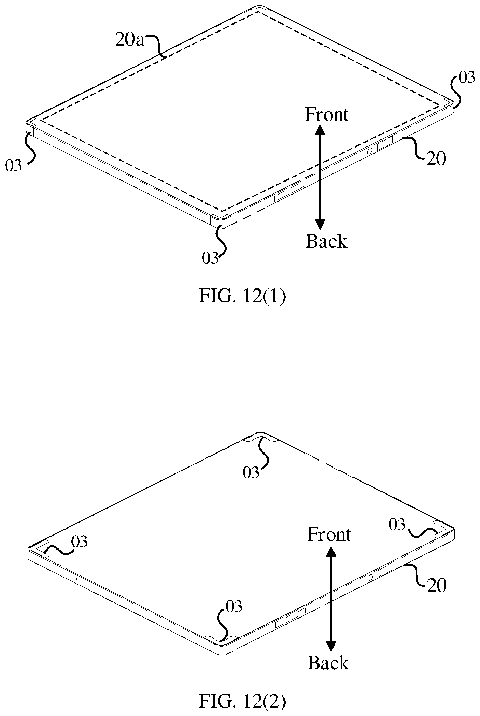

FIG. 12 (1) is a schematic diagram of a movable detector arranged with tags in one embodiment; and FIG. 12(2) is a schematic diagram of a movable detector arranged with tags in one embodiment;

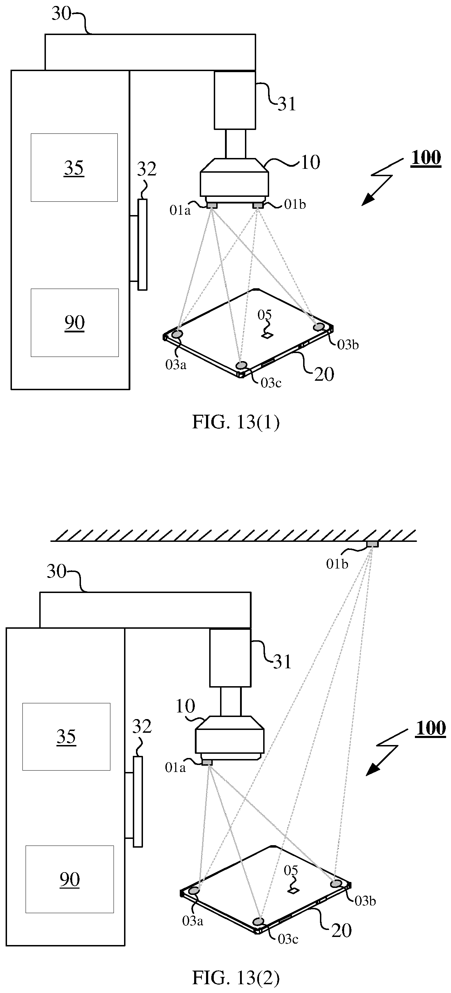

FIG. 13 (1) is a schematic diagram of a radiographic imaging device in one embodiment; and FIG. 13 (2) is a schematic diagram of a radiographic imaging device in one embodiment;

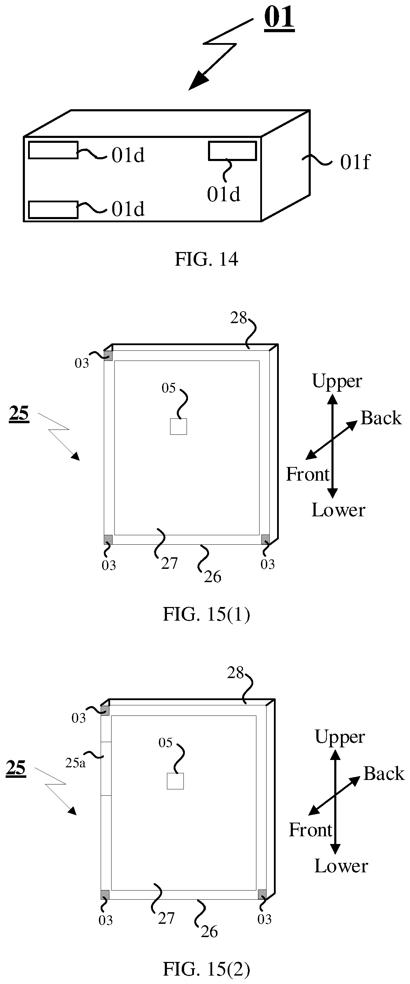

FIG. 14 is a schematic diagram of a base station in one embodiment;
FIG. 15 (1) is a schematic diagram of a movable assistant device in one embodiment; and FIG. 15 (2) is a schematic diagram of a movable assistant device in one embodiment;

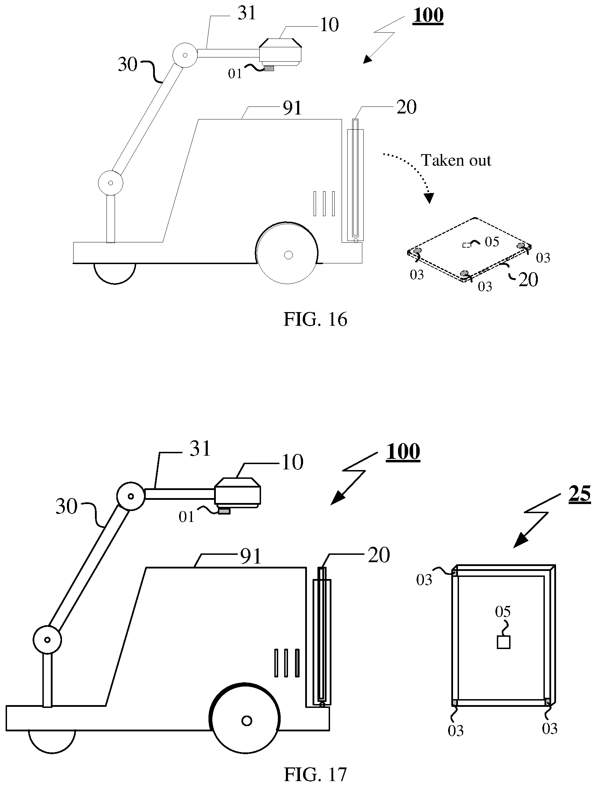

FIG. 16 is a schematic diagram of a radiographic imaging device in one embodiment;
FIG. 17 is a schematic diagram of a radiographic imaging device in one embodiment; and

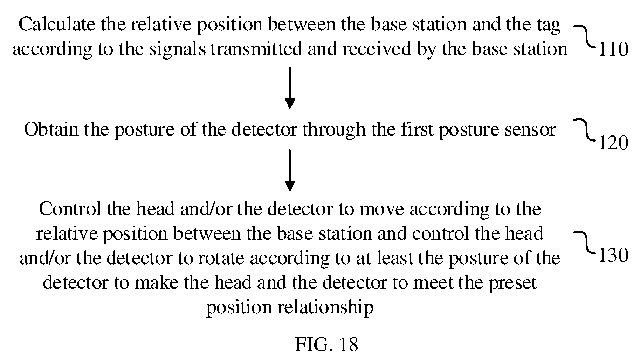

FIG. 18 is a flow diagram of a positioning method for a radiographic imaging device in one embodiment.

### DETAILED DESCRIPTION

The present disclosure will be described in detail below through specific embodiments in combination with the drawings, where similar elements in different embodiments will be assigned with similar reference numbers. In the embodiments below, the detailed description is intended to make the present disclosure to be better understood. However, those skilled in the art can easily recognize that some of the features can be omitted or be replaced by other elements, materials or methods in different situations. In some cases, some operations of the present disclosure will not be shown or described in the specification, which is to avoid the core part of the present disclosure being overwhelmed by too much description. For those skilled in the art, it is not necessary to describe these operations in detail. They can fully understand the operations based on the description in the specification and the general technical knowledge in the field.

In addition, the features, operations or characteristics described in the specification can be combined in any appropriate manner to form various embodiments. The steps or actions in the description of method can also be interchanged or adjusted in a manner that is obvious to those skilled in the art. Therefore, the various sequences in the specification and the drawings are merely for the purpose of clearly describing a certain embodiment, but do not mean necessary sequences, unless it is otherwise specified that a certain sequence must be followed.

The serial numbers of the components herein, such as "first", "second", etc., are merely used to distinguish the objects described and do not have any order or technical meaning. The "connection" and "coupling" herein, unless otherwise specified, include direct and indirect connections (couplings).

In most existing radiographic imaging devices, the detectors are placed in the cassette assembly, which means the detectors cannot be used independently of the cassette assembly. The imaging device can control the movement of the head through driving assembly such as mechanical arms. The cassette assembly is a component that includes a cassette and a bracket. The cassette can house the detectors, and the bracket can support the cassette and even drive the cassette to move. Therefore, the imaging device can easily obtain the positional relationship between the head and the detector through the cassette assembly and the driving assembly, including their spatial positions and postures, etc., thereby making it relatively convenient to achieve the centering or alignment therebetween. For this kind of device, since the detector cannot be used independently of the cassette assembly, the imaging device can only be applied to imaging in a specific body position, but it is impossible to carry out the imaging in a special body position or in various different body positions.

Movable detectors can meet the imaging needs for different body positions. However, since the movable detector can be used without being located in the cassette assembly, but can be in other locations, the imaging device cannot position the detector through the cassette assembly, which affects the position matching between the head and the detector.

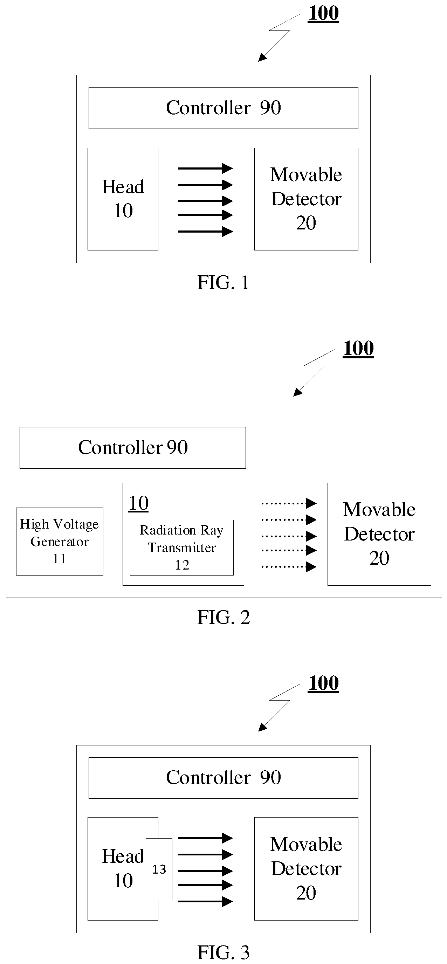

Referring to FIG. 1, in some embodiments of the present disclosure, a radiographic imaging device 100 is provided, which includes a head 10, a movable detector 20 and a controller 90. The head 10 is configured to transmit radioactive radiation rays such as X-radiation rays to the object being examined. The movable detector 20 is configured to receive radioactive radiation rays passing through the object being examined for imaging. In some embodiments, the movable detector 20 can be a movable flat panel detector 20. The detailed description will be shown below.

The head 10 is configured to generate radiation rays, for example, to generate radiation rays under a high voltage signal. In some embodiments, referring to FIG. 2, the radiographic imaging device 100 includes a high voltage generator 11, and the head 10 includes a radiation ray transmitter 12. The high voltage generator 11 is electrically connected to the radiation ray transmitter 12. The high voltage generator 11 is configured to provide a high voltage signal, such as a hundred volts or a thousand volts high voltage, to the radiation ray transmitter 12. The radiation ray transmitter 12 is configured to bombard electrons on the target surface under the high voltage signal to generate radioactive radiation rays such as X-radiation rays. The radiation ray transmitter 12 may be a tube, for example. The high-voltage generator 11 may be arranged inside the head 10. The high-voltage generator may also be arranged in other locations of the radiographic imaging device 100.

In some embodiments, referring to FIG. 3, the head 10 may further include a beam limiter 13. In some embodiments, the beam limiter 13 is configured to define the radiation projection area of the head 10. The radiation projection area can be referred to as irradiation field or irradiation field area.

The movable detector 20 has an important impact on imaging quality. In some embodiments, the movable detector 20 is configured to receive radioactive radiation rays such as X-radiation rays and convert them into electrical signals to complete the image acquisition. Referring to FIG. 4 (1), in one embodiment, the movable detector 20 includes a radiation conversion layer 21 and a TFT matrix layer 22. The radiation conversion layer 21 is configured to convert the radioactive radiation rays such as X-radiation rays into visible light. The radiation conversion layer 21 generally includes a scintillation layer or a fluorescent layer, which is used to convert radiation rays into visible light. Taking the scintillation layer as an example, it can generally be made of scintillation materials, such as cesium iodide (CsI) or gadolinium oxysulfide (GOS), etc. The TFT matrix layer 22 is configured to sense the visible light converted by the radiation conversion layer 21 and convert the visible light into electrical signals for image/data acquisition. In some embodiments, the detector 20 may be a flat-panel detector.

Referring to FIG. 4 (2), in some embodiments, the movable detector 20 may have a communication device 23 which is configured for communication, including data transmission. For example, the communication device 23 may transmit the image data obtained by the detector 20, such as to the controller 90 of the radiographic imaging device 100. The controller 90 may generate an image based on the received image data. Alternatively, the communication device 23 may transmit the image data to the host computer of the radiographic imaging device 100. The host computer can generate an image based on the received image data. In some embodiments, the communication device 23 may be a wireless communication device, such as a Wi-Fi communication device. In some embodiments, the controller 90 may perform control functions or perform processing functions.

In the embodiments of the present disclosure, the imaging needs for different body positions may be satisfied by the utilization of the movable detector 20. The movable detector 20 can be used independently of the cassette assembly. For example, when the person being examined is sitting in a wheelchair, the technician can place the movable detector 20 on the backrest of the wheelchair or under the wheelchair to perform the imaging. For example, for a person being examined lying on a transfer bed, the technician can place the movable detector 20 under the person's body to perform the imaging.

In some embodiments, with the movable detector 20 that is not limited to be placed at a specific location, the radiographic imaging device 100 can perform radiation ray imaging operations in various different body positions, such as the patella axial position, the foot lateral position, the foot anterior-posterior position, the foot oblique position, the hip joint lateral position, the chest anterior-posterior position, the elbow joint lateral position, the elbow joint anterior-posterior position, the abdomen anterior-posterior position, the upper limb front and side position, the cervical spine front position, the cervical spine lateral position, the knee joint lateral position, the tibia-heel position, the ankle joint lateral position, the spine lateral position, the lower limb anterior-posterior position, etc. Therefore, the application scenarios of the X-radiographic imaging device 100 can be effectively expanded.

In some embodiments, the radiographic imaging device 100 may further include a support assembly 30. The support assembly 30 may include a first support element 31. The first support element 31 is connected to the head 10 and is used to support the head 10. FIG. 5 (1) shows an example.

In some embodiments, the radiographic imaging device 100 may further include a driving assembly 35. FIG. 5 (2) shows an example. The driving assembly 35 can drive the head 10 to move. For example, the driving assembly 35 may drive the head 10 to move through the first support element 31. In some embodiments, the driving assembly 35 may drive the head 10 to move in space, for example, to move one-dimensionally, two-dimensionally or three-dimensionally in space. In some embodiments, the driving assembly 35 may rotate the head 10 through the first support element 31, thereby changing the orientation of the head 10.

FIG. 6 shows an example of a mobile radiographic imaging device 100. The mobile radiographic imaging device 100 may include a movable body 91 that includes a body 91a and a movable assembly 91b. The movable assembly 91b can bring the body 91a to move. For example, the movable assembly 91b may include wheels disposed at the lower part of the body 91a. The first support element 31 may be arranged on the movable body 91. The first support element 31 may be a mechanical arm. The driving assembly 35 may control the movement of the mechanical arm through a motor, thereby driving the head 10 to move together. In some embodiments, the movable detector 20 can be conveniently stored in the body 91 when not in use. Since the radiographic imaging device 100 has a movable body 91, it can be moved to the desired location for use. For example, it can be pushed to the operating room, emergency room, ICU ward, neonatal department or isolation area for critically ill patients. In some embodiments, the head 10 can be suspended and movably arranged on the movable body 91, and can be moved in one-dimensional, two-dimensional or three-dimensional directions in space through the first support element 31. The movable detector 20 can be mechanically detached from the movable body 91 for use. For example, the technician can take out the movable detector 20 from the movable body 91. In some embodiments, after receiving an exposure request, the controller 90 controls the head 10 to expose, that is, to transmit radioactive radiation rays such as X-radiation rays to the object being examined, and controls the movable detector 20 to work in conjunction with the head 10 to receive the radioactive radiation rays such as X-radiation rays passing through the object being examined to perform the imaging. After the imaging, the data can be transmitted, e.g., in a wireless manner, to the controller 90 for image processing and subsequent display control.

FIG. 7 (1) shows an example of a fixed radiographic imaging device 100. In some embodiments, the fixed radiographic imaging device 100 may include a column 92, and the first support element 31 may include a slide rail 93 disposed on the column 92. The head 10 is movably disposed on the slide rail 93, and can move at least in one-dimensional direction in space through the slide rail 93. Alternatively, the first support element 31 may further include a one-dimensional or two-dimensional mechanical arm 94, and the head 10 is disposed on the slide rail 93 through the mechanical arm 94, such that the head 10 can move in two or three dimensions in space through the mechanical arm 94 and the slide rail 93. In some embodiments, the head 10 may be driven to rotate through the mechanical arm 94. In some embodiments, the driving assembly 35 controls the movement of the first support element 31 through a motor, thereby driving the head 10 to move. In some embodiments, the radiographic imaging device 100 may further include a bed plate 95 and a bed support element 96 for supporting the bed plate 95. The bed plate 95 can be used to support the object being examined, for example, for the object to lie on. It should be noted that in addition to being provided with the movable detector 20, the radiographic imaging device 100 may also be provided with a vertically arranged detector 97 and a horizontally arranged detector 98. The detectors 97 and 98 may both be wired.

FIG. 7 (2) shows an example of a fixed radiographic imaging device 100. In some embodiments, the radiographic imaging device 100 may include a column 92b, and the head 10 is movably arranged on the column 92b through the first support element 31. For example, the first support element 31 may include a mechanical arm 94a, and the head 10 is arranged on the column 92b through the mechanical arm 94a. In some embodiments, the head 10 movably arranged on the column 92b may be moved in one-dimensional, two-dimensional or three-dimensional directions. In some embodiments, the head may also be rotated. In some embodiments, the driving assembly 35 controls the movement of the first support element 31 through a motor, thereby driving the head 10 to move. For example, the driving assembly 35 controls the movement of the mechanical arm 94a through a motor, thereby driving the head 10 to move. In some embodiments, the radiographic imaging device 100 may further include the bed plate 95 and the bed support element 96 for supporting the bed plate 95. The bed plate 95 can be used to support the object being examined, for example, for the object to lie on. It should be noted that in addition to being provided with the movable detector 20, the radiographic imaging device 100 may also be provided with the vertically arranged detector 97 and the horizontally arranged detector 98. The detectors 97 and 98 may both be wired. The vertically arranged detector 97 may be arranged on the column 92a.

FIG. 7 (3) shows an example of a fixed radiographic imaging device 100. The first support element 31 may include a slide rail 93 arranged on the ceiling. The head 10 is movably arranged on the slide rail 93, and can be moved at least in one-dimensional direction in space through the slide rail 93. In addition, the first support element 31 may also include a one-dimensional or two-dimensional mechanical arm 94, and the head 10 is arranged on the slide rail 93 through the mechanical arm 94, such that the head 10 can be moved in two-dimensional plane or three-dimensional space through the mechanical arm 94 and the slide rail 93. In some embodiments, the head 10 may also be driven to rotate through the mechanical arm 94. In some embodiments, the driving assembly 35 controls the movement of the first support element 31 through a motor, thereby driving the head 10 to move. For example, the driving assembly 35 controls the movement of the mechanical arm 94 through the motor, thereby driving the head 10 to move. In some embodiments, the radiographic imaging device 100 may further include a bed plate 95 and a bed support element 96 for supporting the bed plate 95. The bed plate 95 may be used to support the object being examined, for example, for the object to lie on. It should be noted that, in addition to being provided with the movable detector 20, the radiographic imaging device 100 may also be provided with the vertically arranged detector 97 and the horizontally arranged detector 98. The detectors 97 and 98 may be wired. The vertically arranged detector 97 may be arranged on the column 92.

Referring to FIG. 8. in some embodiments, the support assembly 30 may further include a second support element 32. In some embodiments, the driving assembly 35 can drive the second support element 32 to move.

In some embodiments, the movable detector 20 may be arranged on the second support element 32. When the movable detector 20 is arranged on the second support element 32, the second support element 32 can bring the movable detector 20 to move.

In some embodiments, the radiographic imaging device 100 described herein may include a digital radiography device (DR).

Whether the imaging device 100 is a mobile radiographic imaging device or a fixed radiographic imaging device, the utilization of the movable detector 20 can satisfy the imaging needs of different body positions. However, this also poses a challenge for the position matching between the head 10 and the movable detector 20.

In some embodiments, referring to FIG. 9, the radiographic imaging device 100 includes at least one base station 01 and at least one tag 03. The radiographic imaging device 100 further includes a first posture sensor 05. The base station 01 is configured to transmit signals to the tag and receive signals returned from the tag 03. It should be noted that the positions of the base station 01 and the tag 03 shown in FIG. 9 is merely for illustration, but not intended to be a limitation, which will be further explained below.

The base station 01 and the tag 03 may have a one-to-one or one-to-many relationship. For one base station 01, by transmitting a signal to one tag 03 through this base station 01 and receiving a signal returned from this tag 03, the controller 90 can calculate the relative position between this base station 01 and this tag 03. This way, when we know the spatial position of one of the base station 01 and the tag 03, we can obtain the spatial position of the other of the base station 01 and the tag 03 according to the relative position. In the present disclosure, the spatial position refers to the location of an object in three-dimensional space. The spatial position of an object can be described by XYZ three-dimensional spatial coordinate system. It can be understood that the origin of the XYZ three-dimensional spatial coordinate system may be set at any location according to actual needs. For example, the origin may be a fixed location in space, or it may be the transmission point of the head 10.

In some embodiments, the relative position of an object A and an object B includes the relative distance and the relative angle. Specifically, the relative distance between the object A and the object B may be, considering the object A and the object B as two points, the distance between these two points. The relative angle between the object A and the object B may be, considering the object A and the object B as two points, the angle of a line determined by these two points in space, such as the angle between this line and the X axis, Y axis and/or Z axis in the XYZ three-dimensional coordinate system. In this case, when the relative position between the object A and the object B and the spatial position of one of the object A and the object B are known, the spatial position of the other of the object A and the object B can be determined. Therefore, it is understandable that the relative position between the base station 01 and the tag 03 includes the relative distance and the relative angle. Specifically, the relative distance between the base station 01 and the tag 03 may be, considering the base station 01 and the tag 03 as two points, the distance between these two points. The relative angle between the base station 01 and the tag 03 may be, considering the base station 01 and the tag 03 as two points, the angle of a line determined by these two points in space, such as the angle between this line and the X axis, Y axis and/or Z axis in the XYZ three-dimensional coordinate system. In this case, when the relative position between the base station 01 and the tag 03 and the spatial position of one of the base station 01 and the tag 03 are known, the spatial position of the other one of the base station 01 and the tag 03 can be determined.

In some embodiments, at least one of the base stations 01 is a first base station 01. The first base station 01 may transmit signals to the tag 03 and receive signals returned from the tag 03. The controller 90 may calculate the relative position between the first base station 01 and the tag 03 according to the signals transmitted and received by the first base station 01. It can be understood that the relative position between the first base station 01 and the tag 03 includes the relative distance and the relative angle. The specific meaning of the relative distance and the relative angle is explained above, and it will not be repeated here.

In some embodiments, the first base station 01 and the tag 03 are arranged at the locations where the relative position between the first base station 01 and the tag 03 is able to at least represent the spatial position of the movable detector 20.

In some embodiments, the controller 90 may calculate the spatial position of the movable detector 20 according to the relative position between the first base station 01 and the tag 03.

In some embodiments, the first posture sensor 05 is arranged at the movable detector 20, and configured to measure the posture of the movable detector 20. In some embodiments, the data of the first posture sensor 05 may be transmitted to the controller 90 through, e.g., the communication device 23.

The posture of the movable detector 20 may be the detector's posture in space, such as one or more of its roll angle, pitch angle and yaw angle. The detector's posture in space may be defined by one or more of the roll angle, the pitch angle and the yaw angle of the imaging surface of the movable detector 20, or by one or more of the angles between the imaging surface of the movable detector 20 and the X axis, Y axis and Z axis in the XYZ three-dimensional coordinate system. The face of the movable detector 20 that faces the object being examined or the head 10 during use is the front face of the detector 20. The front face of the detector 20 has an area for receiving radiation rays, which can be referred to as the imaging surface (or imaging surface area) of the detector 20. For example, the area 20a defined by the dashed line in FIG. 12(1) below is an example of the imaging surface area.

In some embodiments, the first posture sensor 05 is arranged at the back face of the detector 20. The back face of the detector 20 is the face opposite to the front face of the detector 20, which will be further described below.

The first posture sensor 05 may also be arranged at other locations that do not affect the detector 20 receiving radiation rays.

In some embodiments, the first posture sensor 05 may include a motion sensor such as a three-axis gyroscope, a three-axis accelerometer or a three-axis electronic compass, etc. In some embodiments, the first posture sensor 05 may be a three-dimensional motion posture measurement device based on MEMS (Micro-ElectroMechanical System) technology.

In some embodiments, the first posture sensor 05 may obtain the temperature-compensated three-dimensional posture and orientation data through an embedded ARM processor.

In some embodiments, the first attitude sensor 05 may utilize the quaternion-based three-dimensional algorithms and data fusion technology to output the zero-drift three-dimensional posture and orientation data represented by quaternions and Euler angles in real time, and may also output nine-axis data including three-axis accelerometer data, three-axis gyroscope data and three-axis magnetic field data.

Therefore, in some embodiments, the posture of the detector 20, such as the roll angle, the pitch angle and the yaw angle, can be obtained through the nine-axis first posture sensor 05.

Moreover, since the controller 90 can drive the head 10 to move by driving the first support element 31 through the driving assembly 35, the controller 90 can obtain the spatial position and even the posture (the orientation of the head 10) of the head 10 according to the driving assembly 35.

In the present disclosure, the posture of the head 10 may be the posture of the head 10 in space, such as one or more of its roll angle, pitch angle and yaw angle. Generally, the radiation rays transmitted by the head 10 is a cone-shaped (e.g., pyramid-shaped) radiation rays, which is caused by the constraint and limitation applied to the radiation rays by the beam limiter 13 of the head 10. The line defined by the vertex of the cone (i.e., the transmission point of the head 10) and the center of the bottom surface of the cone may be referred to as the center line of the cone-shaped radiation rays transmitted by the head 10 (simply referred to as the transmission center line of the head 10). The posture of the head 10 in space may be defined by one or more of the angles between the transmission center line of the head 10 and the X axis, the Y axis and the Z axis in the XYZ three-dimensional coordinate system. The surface parallel to the bottom surface of the cone may be referred to as the irradiation surface of the head 10. The posture of the head 10 in space may also be defined by one or more of the angles between the irradiation surface of the head 10 and the X axis, the Y axis and the Z axis in the XYZ three-dimensional coordinate system.

Please see the details below.

In some embodiments, at least one base station 01, such as the first base station 01 and/or the second base station 01, is arranged in the head 10, for example, in the beam limiter 13 of the head 10. At least one tag 03 is arranged in the movable detector 20, for example, at the corners of the movable detector 20. In the embodiments where both the first base station 01 and the second base station 01 are arranged in the head 10, the distance between the first base station 01 and the second base station 01 is greater than a distance threshold, such that they do not interfere with each other. In some embodiments, the movable detector 20 may be arranged with one tag, two tags or at least three tags.

Since the controller 90 can drive the head 10 to move by driving the first support element 31 through the driving assembly 35, the controller 90 can know the spatial position of the head 10. Moreover, since the base station 01 (for example, the first base station 01) is arranged in the head 10, the controller 90 can know the spatial position of the base station 01 (for example, the first base station 01). In connection with the relative position between the base station 01 (for example, the first base station 01) arranged in the head 10 and the tag 03 arranged in the detector 20, the spatial position of the tag 03 arranged in the detector 20 can be calculated, and therefore the spatial position of the detector 20 can be obtained. For example, the spatial position of the detector 20 may be represented by the spatial position of the tag 03 arranged in the detector 20. Additionally, since the position of the tag 03 arranged in the detector 20 with respect to the detector 20 is known, the spatial position of any location on the detector 20, such as the center of the imaging surface, can be obtained according to the spatial position of the tag 03 arranged in the detector 20. The spatial position of the center of the imaging surface of the detector 20 can represent the spatial position of the detector 20.

In some embodiments, the controller 90 may calculate the spatial position of the movable detector 20 according to the relative position between one base station 01, such as the first base station 01, and at least one tag 03.

In some embodiments, at least one base station 01, such as the first base station 01 and/or the second base station 01, may be arranged in a specific location in the space, such as on the ceiling or in a column 92, etc. At least one tag 03 is arranged in the movable detector 20, for example, at the corner of the movable detector 20. In the embodiments where the first base station 01 and the second base station 01 are respectively arranged in the specific locations in the space, the distance between the base stations is greater than the distance threshold, such that they do not interfere with each other.

Since the base station 01 (for example, the first base station 01) is arranged at a specific location in space, the spatial position of this base station 01 (for example, the first base station 01) is constant. Therefore, the spatial position of this base station 01 (for example, the first base station 01) can be input into the radiographic imaging device 100 in advance, such that the controller 90 can obtain the spatial position of this base station 01 (for example, the first base station 01) and can, in connection with the relative position between this base station 01 (for example, the first base station 01) and the tag 03 arranged in the detector 20, calculate the spatial position of the tag 03 arranged in the detector 20, thereby obtaining the spatial position of the detector 20. For example, the spatial position of the detector 20 may be represented by the spatial position of the tag 03 arranged at the detector 20. Alternatively, since the position of the tag 03 arranged at the detector 20 with respect to the detector 20 is known, the spatial position of any location on the detector 20, such as the center of the imaging surface, can be obtained according to the spatial position of the tag 03 arranged in the detector 20. The spatial position of the center of the imaging surface of the detector 20 can represent the spatial position of the detector 20.

In some embodiments, the controller 90 may calculate the spatial position of the movable detector 20 according to the relative position between one base station 01, such as the first base station 01, and at least one tag 03.

In some embodiments, at least one tag 03 is arranged at the head 10. Similarly, since the controller 90 can obtain the spatial position of the base station 01 (for example, the first base station 01) arranged in a specific location in space, the spatial position of the tag 03 arranged in the head 10 can be calculated according to the relative position between the base station 01 (for example, the first base station 01) and the tag 03 arranged at the head 10 and the spatial position of the base station 01. Therefore, the spatial position of the head 10 can be obtained. For example, the spatial position of the head 10 may be represented by the spatial position of the tag 03 arranged at the head 10. Alternatively, the spatial position of the transmission point of the head 10 may be obtained according to the spatial position of the tag 03 arranged at the head 10. The spatial position of the transmission point of the head 10 may represent the spatial position of the head 10.

In some embodiments, the head 10 is arranged with multiple tags 03.

The spatial position of the head 10 may be represented by the spatial position of any one of the tags 03. It is also possible that the spatial position of the transmission point of the head 10 is calculated according to the spatial position of any one of the tags 03 to represent the spatial position of the head 10. The spatial position of the head 10 may also be calculated according to the spatial positions of these tags 03. For example, the spatial position of the transmission point of the head 10 may be calculated to represent the spatial position of the head 10. These calculations can be completed by the controller 90.

The posture of the head 10 may be obtained according to the multiple tags 03 arranged in the head 10.

In some embodiments, the head 10 is arranged with multiple tags 03, and the controller 90 calculates the posture of the head 10 according to the spatial positions of these tags 03. In some embodiments, at least three tags 03 are arranged in the head 10. In some embodiments, three tags 03 are arranged at three sites of the head 10, and the three sites are not on one straight line. For example, the three sites are located in the beam limiter 13, and the plane defined by the three sites is parallel to the irradiation surface of the head 10. Therefore, in the cast that the head 10 is arranged with at least three tags 03, the spatial position of each tag can be calculated. With three non-collinear points, a plane can be determined, such as the irradiation surface of the head 10. Therefore, the posture of the head 10 can be calculated by the controller 90.

In some embodiments, referring to FIG. 10, the head 10 may be arranged with a second posture sensor 07 configured to measure the posture of the head 10.

In some embodiments, the second posture sensor 07 may include a motion sensor such as a three-axis gyroscope, a three-axis accelerometer or a three-axis electronic compass, etc. In some embodiments, the second posture sensor 07 may be a three-dimensional motion posture measurement device based on MEMS (Micro-ElectroMechanical System) technology.

In some embodiments, the second posture sensor 07 may obtain the temperature-compensated three-dimensional posture and orientation data through an embedded ARM processor.

In some embodiments, the second posture sensor 07 may utilize the quaternion-based three-dimensional algorithms and data fusion technology to output the zero-drift three-dimensional posture and orientation data represented by quaternions and Euler angles in real time, and may also output nine-axis data including three-axis accelerometer data, three-axis gyroscope data and three-axis magnetic field data.

Therefore, in some embodiments, the posture of the head 10, such as the roll angle, the pitch angle and the yaw angle, can be obtained through the nine-axis second posture sensor 07.

In some embodiments, the movable detector 20 is arranged with multiple tags 03.

The spatial position of the movable detector 20 may be represented by the spatial position of any one of the tags 03. Alternatively, the spatial position of the center of the imaging surface of the detector 20 may be calculated according to the spatial position of any one of the tags 03 to represent the spatial position of the detector 20. The spatial position of the movable detector 20 may also be calculated according to the spatial positions of these tags 03. For example, the spatial position of the center of the imaging surface of the movable detector 20 may be calculated according to the spatial positions of the tags 03 to represent the spatial position of the movable detector 20. These calculations can be completed by the controller 90.

FIG. 11 (1) shows an example where tags 03 are arranged at two opposite corners of the movable detector 20, such as tag 03a and tag 03b. The controller 90 can calculate the spatial position of the center of the movable detector 20 according to the spatial positions of tag 03a and tag 03b. FIG. 11 (2) shows an example where tags 03 are arranged at three corners of the movable detector 20, such as tag 03a, tag 03b and tag 03c. The controller 90 can calculate the spatial position of the center of the movable detector 20 according to the spatial positions of tag 03a, tag 03b, and tag 03c. FIG. 11 (3) shows an example where the tags 03 are arranged at four corners of the movable detector 20, such as tag 03a, tag 03b, tag 03c, and tag 03d. The controller 90 can calculate the spatial position of the center of the movable detector 20 according to the spatial positions of tags 03a, 03b, 03c, and 03d.

The posture of the movable detector 20 may be obtained according to the multiple tags 03 arranged at the movable detector 20.

In some embodiments, the movable detector 20 is arranged with multiple tags 03. The controller 90 calculates the posture of the movable detector 20 according to the spatial positions of these multiple tags 03. In some embodiments, the movable detector 20 is arranged with at least three tags 03. In some embodiments, three tags 03 are arranged at three sites of the movable detector 20, and the three sites are not on one straight line. For example, the three sites are arranged at three corners of the movable detector 20. FIG. 11 (2) and FIG. 11 (3) show two examples. In the example shown in FIG. 11 (2), the spatial position of the tag 03a can be calculated according to the relative position between the first base station 01 and the tag 03a, the spatial position of the tag 03b can be calculated according to the relative position between the first base station 01 and tag the 03b, and the spatial position of the tag 03c can be calculated according to the relative position between the first base station 01 and the tag 03c. In the example shown in FIG. 11 (3), the spatial position of the tag 03a can be calculated according to the relative position between the first base station 01 and the tag 03a, the spatial position of the tag 03b can be calculated according to the relative position between the first base station 01 and tag the 03b, the spatial position of the tag 03c can be calculated according to the relative position between the first base station 01 and the tag 03c, and the spatial position of the tag 03d can be calculated according to the relative position between the first base station 01 and the tag 03d. Therefore, in the case that the movable detector 20 is arranged with at least three tags 03, the spatial position of each tag can be calculated. With the three non-collinear points, a plane can be determined, such as the plane where the imaging surface of the detector 20 is located. Therefore, the posture of the movable detector 20 can be calculated by the controller 90.

In some embodiments, the controller 90 calculates the spatial position of the movable detector 20 according to the relative position between the base station 01, such as the first base station 01, and at least one tag 03.

In some embodiments, the controller 90 calculates the posture of the movable detector 20 according to the relative positions between the base station 01, such as the first base station 01, and at least three tag 03.

In some embodiments, the detector 20 and the tag 03 arranged at the detector 20 are assembled as a single unit, thereby forming the overall contour structure of the detector 20. FIG. 12(1) and FIG. 12(2) show two examples. The detector 20 has a front face, a back face and side faces. As mentioned above, the face of the movable detector 20 that faces the object being examined or the head 10 during use is the front face. The front face of the detector 20 has an area for receiving radiation rays, which can be referred to as the imaging surface (or imaging surface area) of the detector 20. The area 20a defined by the dashed line in the figure is an example of the imaging surface area. The back face of the detector 20 is the face that is opposite to the front face of the detector 20 in the front-rear direction of the detector 20 in the figure. The side faces of the detector 20 are the faces connecting the back face and the front face. The tags 03 may be arranged at the side face of the detector 20 at the corner, as shown in FIG. 12(1). The tags 03 may also be arranged at the front face at the corner, as shown in FIG. 12(2).

In some embodiments, the tags 03 may be arranged outside the imaging surface area of the detector 20.

In some embodiments, the detector 20 includes a radiation conversion layer 21 and a TFT matrix layer 22 in sequence from front to back. The first posture sensor 05 may be arranged inside the detector 20, and arranged behind the TFT matrix layer 22 in the front-back direction. This way, the first posture sensor 05 does not affect the detector 20 receiving the radiation rays.

It can be seen that the spatial positions and postures of the head 10 and the movable detector 20 can be obtained through various schemes. Furthermore, calibration can be performed according to the spatial positions and postures of the head 10 and the movable detector 20 obtained through the different schemes, which will be described in details below.

In some embodiments, the first base station 01 is arranged at the head 10. The movable detector 20 is provided with one or more tags 03, such as three or more tags 03. The movable detector 20 is provided with the first posture sensor 05.

The controller 90 may calculate the spatial position and/or the posture of the movable detector 20 according to the relative position between the first base station 01 and the tag 03. In the embodiments where the controller 90 calculates the posture of the movable detector 20 according to the relative position between the first base station 01 and the tag 03, the controller 90 may also calibrate the posture of the detector 20 obtained by the first posture sensor 05 according to the posture calculated.

Furthermore, in the embodiments where the second base station 01 is arranged at the head 10 or at a specific location in space, the controller 90 may calculate the spatial position and/or posture of, for example, the movable detector 20 according to the relative position between the second base station 01 and the tag 03 at the detector 20. Therefore, the controller 90 may calibrate the spatial position of the movable detector 20 calculated through the first base station 01 using the spatial position of the movable detector 20 calculated through the second base station 01, and the controller 90 may also calibrate the posture of the movable detector 20 obtained by the first posture sensor 05 using the posture of the movable detector 20 calculated through the second base station 01.

FIG. 13(1) shows an example. For easy distinction, in FIG. 12(1), 01a represents the first base station arranged at the head 10, 01b represents the second base station arranged at the head 10, and 03a, 03b and 03c represent three tags arranged at the movable detector 20. The grey solid lines in the figure represent the transmission and reception signals between the first base station 01a and the three tags 03a, 03b and 03c, and the grey dashed lines in the figure represent the transmission and reception signals between the second base station 01b and the three tags 03a, 03b, and 03c. The relative position between the first base station 01a and the tag 03a can be calculated according to the signals transmitted and received by the first base station 01a to and from the tag 03a. The relative position between the first base station 01a and the tag 03b can be calculated according to the signals transmitted and received by the first base station 01a to and from the tag 03b. The relative position between the first base station 01a and the tag 03c can be calculated according to the signals transmitted and received by the first base station 01a to and from the tag 03c. Therefore, according to the three relative positions, the spatial position of the movable detector 20 can be calculated (let's call it the spatial position of the movable detector 20 calculated through the first base station 01a), and the posture of the movable detector 20 can also be calculated (let's call it the posture of the movable detector 20 calculated through the first base station 01a). Similarly, the relative position between the second base station 01b and the tag 03a can be calculated according to the signals transmitted and received by the second base station 01b to and from the tag 03a. The relative position between the second base station 01b and the tag 03b can be calculated according to the signals transmitted and received by the second base station 01b to and from the tag 03b. The relative position between the second base station 01b and the tag 03c can be calculated according to the signals transmitted and received by the second base station 01b to and from the tag 03c. Therefore, according the three relative positions, the spatial position of the movable detector 20 can be calculated (let's call it the spatial position of the movable detector 20 calculated through the second base station 01b), and the posture of the movable detector 20 can also be calculated (let's call it the posture of the movable detector 20 calculated through the second base station 01b). Furthermore, the spatial position of the movable detector 20 calculated through the first base station 01a can be calibrated using the spatial position of the movable detector 20 calculated through the second base station 01b, and the posture of the movable detector 20 obtained by the first posture sensor 05 can be calibrated using one or both of the posture of the movable detector 20 calculated through the second base station 01b and the posture of the movable detector 20 calculated through the first base station 01a.

In the embodiments where the second base station 01 is arranged at a specific location in space, the head 10 may also be provided with one or more tags 03. The controller 90 may calculate the spatial position and/or the posture of the head 10 according to the relative position between the second base station 01 and the tag 03 arranged at the head 10. Therefore, the controller 90 may calibrate the spatial position of the head 10 obtained by the controller 90 through the driving assembly 35 using the spatial position of the head 10 calculated through the second base station 01, and the controller 90 may also calibrate the posture of the head 10 obtained by the controller 90 through the driving assembly 35 using the posture of the head 10 calculated through the second base station 01.

In the embodiments where the second base station 01 is arranged at a specific location in space, the head 10 may be provided with one or more tags 03, and the head 10 may further be provided with a second posture sensor 07. The controller 90 may calculate the spatial position and/or the posture of the head 10 according to the relative position between the second base station 01 and the tag 03 arranged at the head 10. The controller 90 may obtain the posture of the head 10 through the second posture sensor 07. Therefore, the controller 90 may calibrate the spatial position of the head 10 obtained by the controller 90 through the driving assembly 35 using the spatial position of the head 10 calculated through the second base station 01. The controller 90 may calibrate the posture of the head 10 obtained by the second posture sensor 07 using the posture of the head 10 calculated through the second base station 01. The controller 90 may also calibrate the posture of the head 10 obtained by the controller 90 through the driving assembly 35 using the posture of the head 10 obtained by the second posture sensor 07.

In some embodiments, the first base station 01 is arranged at a specific location in space. The movable detector 20 is provided with one or more tags 03, such as three or more tags 03. The movable detector 20 is provided with a first posture sensor 05.

The controller 90 may calculate the spatial position and/or the posture of the movable detector 20 according to the relative position between the first base station 01 and the tag 03. In the embodiments where the controller 90 calculates the posture of the movable detector 20 according to the relative position between the first base station 01 and the tag 03, the controller 90 may also calibrate the posture of the detector 20 obtained by the first posture sensor 05 according to the posture calculated.

Furthermore, the head 10 may be provided with one or more tags 03. The controller 90 may calculate the spatial position and/or the posture of the head 10 according to the relative position between the first base station 01 and the tag 03 arranged at the head 10. Therefore, the controller 90 may calibrate the spatial position of the head 10 obtained by the controller 90 through the driving assembly 35 using the spatial position of the head 10 calculated through the first base station 01, and the controller 90 may also calibrate the posture of the head 10 obtained by the controller 90 through the driving assembly 35 using the posture of the head 10 calculated through the first base station 01.

In the embodiments where the second base station 01 is arranged at a specific location in space, the controller 90 may calculate the spatial position and/or the posture of the movable detector 20 according to the relative position between the second base station 01 and the tag 03 arranged at the detector 20. Therefore, the controller 90 may calibrate the spatial position of the movable detector 20 calculated through the first base station 01 using the spatial position of the movable detector 20 calculated through the second base station 01, and the controller 90 may also calibrate the posture of the movable detector 20 obtained by the first posture sensor 05 using the posture of the movable detector 20 calculated through the second base station 01.

FIG. 13(2) shows an example. For easy distinction, in the figure, 01a represents the first base station arranged in the head 10, 01b represents the second base station arranged on the ceiling, and 03a, 03b and 03c represent three tags arranged at the movable detector 20. The grey solid line in the figure shows the transmission and reception signals between the first base station 01a and the three tags 03a, 03b and 03c, and the grey dashed line in the figure shows the transmission and reception signals between the second base station 01b and the three tags 03a, 03b and 03c. The relative position between the first base station 01a and the tag 03a can be calculated according to the signals transmitted and received by the first base station 01a to and from the tag 03a. The relative position between the first base station 01a and the tag 03b can be calculated according to the signals transmitted and received by the first base station 01a to and from the tag 03b. The relative position between the first base station 01a and the tag 03c can be calculated according to the signals transmitted and received by the first base station 01a to and from the tag 03c. Therefore, according to the three relative positions, the spatial position of the movable detector 20 can be calculated (let's call it the spatial position of the movable detector 20 calculated through the first base station 01a), and the posture of the movable detector 20 can also be calculated (let's call it the posture of the movable detector 20 calculated through the first base station 01a). Similarly, the relative position between the second base station 01b and the tag 03a can be calculated according to the signals transmitted and received by the second base station 01b to and from the tag 03a. The relative position between the second base station 01b and the tag 03b can be calculated according to the signals transmitted and received by the second base station 01b to and from the tag 03b. The relative position between the second base station 01b and the tag 03c can be calculated according to the signals transmitted and received by the second base station 01b to and from the tag 03c. Therefore, according to the three relative positions, the spatial position of the movable detector 20 can be calculated (let's call it the spatial position of the movable detector calculated through the second base station 01b), and the posture of the movable detector 20 can also be calculated (let's call it the posture of the movable detector 20 calculated through the second base station 01b). Furthermore, the spatial position of the movable detector 20 calculated through the first base station 01a can be calibrated using the spatial position of the movable detector 20 calculated through the second base station 01b, and the posture of the detector obtained by the first posture sensor 05 can be calibrated using one or both of the posture of the movable detector 20 calculated through the second base station 01b and the posture of the movable detector 20 calculated through the first base station 01a.

In the embodiments where the second base station 01 is arranged at a specific location in space and the head 10 is provided with one or more tags 03, the controller 90 can calculate the spatial position and/or the posture of the head 10 according to the relative position between the second base station 01 and the tag 03 arranged at the head 10. Therefore, the controller 90 may calibrate the spatial position of the head 10 obtained by the controller 90 through the driving assembly 35 using the spatial position of the head 10 calculated through the second base station 01. Alternatively, the controller 90 may calibrate the spatial position of the head 10 calculated through the first base station 01 using the spatial position of the head 10 calculated through the second base station 01. The controller 90 may also calibrate the posture of the head 10 obtained by the controller 90 through the driving assembly 35 using the posture of the head 10 calculated through the second base station 01. Alternatively, the controller 90 may calibrate the posture of the head 10 calculated through the first base station 01 using the posture of the head 10 calculated through the second base station 01. Alternatively, the controller 90 may calibrate the posture of the detector 20 obtained by the first posture sensor 05 using the posture of the head 10 calculated through the second base station 01.

The embodiments where the base station 01 is arranged at the head 10 or at a specific location in space have been described above. In other embodiments, the base station 10 may be arranged in the movable detector 20, which will be explained in detail below.

In some embodiments, at least one tag 03 is arranged at the head 10, such as on the beam limiter 13 of the head 10. At least one base station 01, such as the first base station 01, is arranged in the movable detector 20, such as at the corner of the movable detector 20.

Since the controller 90 can drive the head 10 to move by driving the first support element 31 through the driving assembly 35, the controller 90 can know the spatial position of the head 10. Moreover, since the tag 03 is arranged at the head 10, the spatial position of the tag 03 is known. Therefore, using the relative position between the tag 03 arranged at the head 10 and the base station 01 (for example, the first base station 01) arranged at the detector 20, the spatial position of the base station 01 (for example, the first base station 01) arranged at the detector 20 can be calculated. Therefore, the spatial position of the detector 20 can be obtained. For example, the spatial position of the base station 01 (for example, the first base station 01) can be used to represent the spatial position of the detector 20. Alternatively, since the position of the base station 01 (for example, the first base station 01) arranged at the detector 20 with respect to the detector 20 is known, the spatial position of any location on the detector 20, such as the center of the imaging surface, can be obtained according to the spatial position of the base station 01 (for example, the first base station 01) arranged at the detector 20. The spatial position of the center of the imaging surface of the detector 20 can be used to represent the spatial position of the detector 20.

In some embodiments, the controller 90 calculates the spatial position of the movable detector 20 according to the relative position between one tag 03 and at least one base station 01, such as the first base station 01.

In some embodiments, at least one tag 03 is arranged at a specific location in the space, such as the ceiling or the column 92, etc. At least one base station 01 (for example, the first base station 01) is arranged in the movable detector 20, e.g., at the corner of the movable detector 20.

Since the tag 03 is arranged in a specific location in the space, its spatial position is constant. Therefore, the spatial position of the tag 03 can be input into the radiographic imaging device 100 in advance. The controller 90 can obtain the spatial position of the tag 03, and calculate the spatial position of the base station 01 (for example, the first base station 01) arranged in the detector 20 according to the relative position between the tag 03 and the base station 01 (for example, the first base station 01) arranged in the detector 20 and the spatial position of the tag 03. Therefore, the spatial position of the detector 20 can be obtained. For example, the spatial position of the detector 20 can be represented by the spatial position of the base station 01 (for example, the first base station 01) arranged in the detector 20. Alternatively, since the position of the base station 01 arranged in the detector 20 (e.g., the first base station 01) with respect to the detector 20 is known, the spatial position of any location on the detector 20, such as the center of the imaging surface, can be obtained according to the spatial position of the base station 01 arranged in the detector 20 (e.g., the first base station 01). The spatial position of the center of the imaging surface of the detector 20 can represent the spatial position of the detector 20.

In some embodiments, the controller 90 calculates the spatial position of the movable detector 20 according to the relative position between a tag 03 and at least one base station 01, such as the first base station 01.

In some embodiments, at least one base station 01 (e.g., the first base station 01) is arranged in the head 10. Similarly, since the controller 90 can obtain the spatial position of the tag 03 arranged at a specific location in space, the spatial position of the base station 01 arranged in the head 10 (e.g., the first base station 01) can be calculated according to the relative position between the tag 03 and the base station 01 arranged in the head 10 (e.g., the first base station 01) and the spatial position of the tag 03. Therefore, the spatial position of the head 10 can be obtained. For example, the spatial position of the base station 01 arranged in the head 10 (e.g., the first base station 01) can be used to represent the spatial position of the head 10. Alternatively, the spatial position of the transmission point of the head 10 may be obtained according to the spatial position of the base station 01 arranged in the head 10 (e.g., the first base station 01). The spatial position of the transmission point of the head 10 may be used to represent the spatial position of the head 10.

In some embodiments, the head 10 is arranged with multiple base stations 01 (for example, the first base station 01).

The spatial position of the head 10 can be represented using the spatial position of any one of the multiple base stations 01 (for example, the first base station 01). Alternatively, the spatial position of the transmission point of the head 10 can be calculated according to the spatial position of any one of the base stations 01 (for example, the first base station 01) to represent the spatial position of the head 10. Alternatively, the spatial position of the head 10 can be calculated according to the spatial positions of these base stations 01 (for example, the first base station 01). For example, the spatial position of the transmission point of the head 10 can be calculated to represent the spatial position of the head 10. These calculations can be done by the controller 90.

The posture of the head 10 may be obtained through the multiple base stations 01 arranged at the head 10 (for example, the first base station 01).

In some embodiments, the head 10 is arranged with multiple base stations 01 (e.g., the first base station 01), and the controller 90 calculates the posture of the head 10 according to the spatial positions of the multiple base stations 01 (e.g., the first base station 01). In some embodiments, the head 10 is arranged with at least three base stations 01 (for example, the first base station 01). In some embodiments, the three base stations 01 (for example, the first base station 01) are arranged at three sites on the head 10. The three sites are not on one straight line. For example, the three sites are arranged in the beam limiter 13. The plane defined by the three sites is parallel to the irradiation surface of the head 10. Therefore, in the case that the head 10 is arranged with at least three base stations 01 (for example, the first base station 01), it is possible to calculate the spatial position of each base station 01 (for example, the first base station 01). With the three non-collinear points, a plane can be determined, such as the irradiation surface of the head 10. Therefore, the controller 90 can obtain the posture of the head 10.

In some embodiments, the head 10 is provided with the second posture sensor 07 configured to measure the posture of the head 10.

In some embodiments, the movable detector 20 is arranged with multiple base stations 01 (for example, the first base station 01).

The spatial position of the movable detector 20 can be represented using the spatial position of any one of the base stations 01 (for example, the first base station 01). Alternatively, the spatial position of the center of the imaging surface of the detector 20 can be calculated according to the spatial position of any one of the base stations 01 (for example, the first base station 01) to represent the spatial position of the detector 20. The spatial position of the movable detector 20 can also be calculated according to the spatial positions of these base stations 01 (for example, the first base station 01). For example, the spatial position of the center of the imaging surface of the movable detector 20 can be calculated to represent the spatial position of the movable detector 20. These calculations can be completed by the controller 90.

The posture of the movable detector 20 can be obtained through the multiple base stations 01 (for example, the first base station 01) arranged at the movable detector 20.

In some embodiments, the movable detector 20 is arranged with multiple base stations 01 (e.g., the first base station 01), and the controller 90 calculates the posture of the movable detector 20 according to the spatial positions of the multiple base stations 01 (e.g., the first base station 01). In some embodiments, the movable detector 20 is arranged with at least three base stations 01 (for example, the first base station 01). In some embodiments, the three base stations 01 (for example, the first base station 01) are arranged at three sites on the movable detector 20. The three sites are not on one straight line. For example, the three sites are arranged at three corners of the movable detector. Therefore, in the case that the movable detector 20 is arranged with at least three base stations 01 (for example, the first base station 01), it is possible to calculate the spatial position of each base station 01 (for example, the first base station 01). With the three non-collinear points, a plane can be determined, such as the imaging surface of the movable detector 20. Therefore, the controller 90 can obtain the posture of the movable detector 20.

In some embodiments, the controller 90 calculates the spatial position of the movable detector 20 according to the relative position between one tag 03 and at least one base station 01, such as the first base station 01.

In some embodiments, the controller 90 calculates the posture of the movable detector 20 according to the relative positions of one tag 03 and at least three base stations 01, for example, at least three first base stations 01.

In some embodiments, the first posture sensor 05 is arranged at the movable detector 20 and configured to measure the posture of the movable detector 20.

It should be noted that the location of the controller 90 can be designed based on the user's needs, and the specific settings and installation location of the controller 90 will not be limited in the present disclosure. In addition, the controller 90 in the present disclosure may have a control function, which can control other components to perform corresponding operations. For example, the controller 90 can be configured to control the driving assembly 35 to drive the head 10 to move, control the display to perform display operations, or control components with data processing and computing functions, such as processors, to perform data processing and computing. The controller 90 may also have data calculation and processing functions itself, such as calculating the relative position according to the signals transmitted and received by the base station 01, etc.

The base station 01 will be described below in conjunction with FIG. 14.

In some embodiments, the base station 01 (e.g., the first base station 01, or the second base station 01) transmits the signal to the tag 03 and receives the signal returned from the tag 03 through its antenna 01d. The controller 90 can position the tag 03 according to the Phase Difference of Arrival (PDOA) algorithm, thereby obtaining the relative position between the base station 01 and the tag 03.

In some embodiments, the base station 01 (for example, the first base station 01, or the second base station 01) may have at least two antennas Old for transmitting and receiving signals, such as the first antenna 01d and the second antenna 01d. In some embodiments, the controller 90 calculates the first relative distance between the first antenna 01d and the tag 03 according to the signals transmitted and received by the first antenna 01d, which can be obtained according to the flight time of the signal. In some embodiments, the controller 90 calculates the first signal phase difference according to the signals transmitted and received by the first antenna 01d and the signals transmitted and received by the second antenna 01d, where the first signal phase difference is the phase difference between the signals received by the first antenna 01d and the signals received by the second antenna 01d. In some embodiments, the controller 90 calculates the relative position between the base station 01 and the tag 03 at least according to the first relative distance and the first signal phase difference. For example, the controller 90 calculates the first azimuth angle of the tag 03 according to the first signal phase difference and the distance between two antennas 01d (the first antenna 01d and the second antenna 01d). The first azimuth angle may be the relative angle between the base station 01 and the tag 03. The first relative distance may be the relative distance between the base station 01 and the tag 03.

In some embodiments, the base station 01 (for example, the first base station 01, or the second base station 01) may have three antennas 01d for transmitting and receiving signals, such as the first antenna 01, the second antenna 01d, and the third antenna 01d. In some embodiments, the controller 90 calculates the first relative distance between the first antenna 01d and the tag 03 according to the signals transmitted and received by the first antenna 01d, which can be obtained according to the flight time of the signal. In some embodiments, the controller 90 calculates the first signal phase difference according to the signals transmitted and received by the first antenna 01d and the signals transmitted and received by the second antenna 01d, where the first signal phase difference is the phase difference between the signals received by the first antenna 01d and the signals received by the second antenna 01d. In some embodiments, the controller 90 calculates the second signal phase difference according to the signals transmitted and received by the first antenna 01d and the signals transmitted and received by the third antenna 01d, where the second signal phase difference is the phase difference between the signals received by the first antenna 01d and the signals received by the third antenna 01d. In some embodiments, the controller 90 calculates the relative position between the base station 01 and the tag 03 at least according to the first relative distance, the first signal phase difference and the second signal phase difference. For example, the controller 90 calculates the first azimuth angle of the tag 03 according to the first signal phase difference and the distance between the two antennas 01d (the first antenna 01d and the second antenna 01d), and calculates the second azimuth angle of the tag 03 according to the second signal phase difference and the distance between the two antennas 01d (first antenna 01d and the third antenna 01d). The first azimuth angle and the second azimuth angle may be the relative angle between the base station 01 and the tag 03, and the first relative distance may be the relative distance between the base station 01 and the tag 03.

In some embodiments, the first azimuth angle is the angle of the connection line connecting the base station 01 and the tag 03 with respect to the XOY plane. In some embodiments, the second azimuth angle is the horizontal azimuth angle of the connection line connecting the base station 01 and the tag 03, that is, the angle between the projection of the connection line connecting the base station 01 and the tag 03 on the XOY plane and the Y axis.

In some embodiments, the three antennas 01d for transmitting and receiving signals of the base station 01 (for example, the first base station 01, or the second base station 01) are arranged at three sites of the base station 01, and the three sites are not on one straight line. In some embodiments, the three sites are arranged at the three vertices of a right triangle.

In some embodiments, the first antenna 01d above may be arranged at the vertex where the right angle of the right triangle is located.

FIG. 14 shows an example, where the base station 01 includes a body 01f and three antennas 01d arranged on the same side of the body 01f.

In some embodiments, the first base station 01 and the second base station 01 are the same.

In some embodiments, the base station 01 is an Ultra Wide Band (UWB) type base station, and the tag 03 is an Ultra Wide Band (UWB) type tag.

Ultra-wide band technology is a wireless personal local area network communication technology that features low power consumption and high-speed transmission. It is suitable for wireless communication applications that require high-quality services and can be used in fields such as wireless personal local area networks (WPANs), home network connections, and short-range radar. Instead of using continuous sine waves, it uses pulse signals for transmission. Ultra-wide band refers to ultra-wide band pulses, which are pulses with a pulse width in the range of nanoseconds to picoseconds. Unlike the continuous carrier wave method commonly used in communication, UWB uses extremely short pulse signals to transmit data. The bandwidth occupied by these pulses even reaches several GHz, so the maximum data transmission rate can reach several hundred Mbps. Because it uses extremely short pulses, when communicating at high speed, the transmission power of UWB devices is very small, and is only a few percent of that of current continuous carrier systems. Therefore, Ultra-Wide band has the characteristics of high spatial resolution, strong anti-interference, fast transmission speed, and low cost and power consumption. Using ultra-wide band type base stations and ultra-wide band type tags for short-distance communication, accurately the relative position can be accurately obtained, thereby obtaining the spatial position and posture of the movable detector 20.

How to obtain the spatial position and posture of the detector 20 through the base station 01 and the tag 03 have been described above.

In some embodiments, the controller may prompt the user according to the relative position between the base station 01 (such as the first base station 01) and the tag 03 and/or the posture of the detector, for example, by generating information for display and/or audio playback. The prompt information may be one or more of the following: the spatial position of the detector 20, the posture of the detector 20, the distance between the detector 20 and the head 10, the distance between the head 10 and the imaging surface of the detector 20, the distance between the head 10 and the first straight line, the angle between the irradiation surface of the head 10 and the imaging surface of the detector 20, the indicator indicating the direction and/or distance in which the user moves the head 10, the indicator indicating the angle or direction to which the user turns the head 10, and so on. With these prompts, the user can control the movement of the head 10 such that the head 10 and the detector 20 meet the preset position relationship.

The first straight line mentioned above is a straight line that is vertical to and passes through the center of the imaging surface of the detector 20.

In some embodiments, the preset position relationship between the head 10 and the detector 20 may include: the distance between the head 10 and the imaging surface of the detector 20 satisfies a first distance requirement, and/or, the distance between the head 10 and the first straight line satisfies a second distance requirement, where the first straight line is a straight line that is vertical to and passes through the center of the imaging surface of the detector.

In some embodiments, the preset position relationship between the head 10 and the detector 20 may include: the angle between the irradiation surface of the head 10 and the imaging surface of the detector 20 satisfies an angle requirement.

The preset position relationship will be described in details below.

In some embodiments, the controller 90 may control the head 10 and the detector 20 to satisfy the preset position relationship through the driving assembly 35.

In some embodiments, the controller 90 may control the driving assembly 35 to drive the head 10 and/or the detector 20 to move according to the relative position between the base station 01 (such as the first base station 01) and the tag 03 (such as the tag 03 arranged at the detector 20), and control the driving assembly 35 to drive the head 10 and/or the detector 20 to rotate according to at least the posture of the detector 20 (such as the posture obtained through the first posture sensor 05), to make the head 10 and the detector 20 to satisfy the preset position relationship.

In some embodiments, the preset position relationship between the head 10 and the detector 20 may include: the distance between the head 10 and the imaging surface of the detector 20 satisfies a first distance requirement, and/or, the distance between the head 10 and the first straight line satisfies a second distance requirement, where the first straight line is a straight line that is vertical to and passes through the center of the imaging surface of the detector.

In some embodiments, the first distance requirement may be that the distance is less than a first distance, where the first distance may be, for example, 1.5 meters, 1.2 meters, 1.1 meters, or 1 meter. In some embodiments, the first distance requirement may be that the distance is within a preset range, such as between 0.8 meters and 1.2 meters, between 0.9 meters and 1.1 meters, and so on.

In some embodiments, the second distance requirement may be that the distance is less than a second distance. The second distance may be, for example, 0.3 meters, 0.2 meters, 0.1 meters, 0.05 meters, and so on.

In some embodiments, the controller 90 calculates the spatial position of the detector 20 according to the relative position between the base station 01 (such as the first base station 01) and the tag 03 arranged at the detector 20. The controller 90 acquires the spatial position of the head 10 and, according to the spatial position of the head 10 and the spatial position of the detector 20, controls the driving assembly 35 to drive the head 10 and/or the detector 20 to move to make the head 10 and the detector 20 to satisfy the preset position relationship, for example, to satisfy the first distance requirement and/or the second distance requirement.

In some embodiments, the controller 90 may obtain the spatial position of the head 10 through the driving assembly 35. In the embodiments where the first base station 01 is arranged at a specific location in space and the head 10 is provided with one or more tags 03, the controller 90 may calculate the spatial position of the head 10 through the relative position between the first base station 01 and the tag 03 arranged at the head 10.

In some embodiments, the preset position relationship between the head 10 and the detector 20 may include: the angle between the irradiation surface of the head 10 and the imaging surface of the detector 20 satisfies the angle requirement.

In some embodiments, the angle requirement may be that the angle is less than a first angle. The first angle may be, for example, 10 degrees, 8 degrees, 5 degrees, 2 degrees, or 1 degree. In some embodiments, the angle requirement may be that the irradiation surface of the head 10 and the imaging surface of the detector 20 are parallel to each other, that is, the angle is 0 degrees.

In some embodiments, the controller 90 obtains the posture of the head 10 and, according to the posture of the head 10 and the posture of the detector 20 (such as the posture obtained through the first posture sensor 05), controls the driving assembly 35 to drive the head 10 and/or the detector 20 to rotate to make the head 10 and the detector 20 meet the angle requirement.

In some embodiments, the controller 90 may obtain the posture of the head 10 through the driving assembly 35. In the embodiments where the first base station 01 is arranged at a specific location in space and the head 10 is provided with one or more tags 03, the controller 90 may calculate the posture of the head 10 through the relative position between the first base station 01 and the tag 03 arranged the head 10. In the embodiments where the head 10 is provided with the second posture sensor 07, the controller 90 may obtain the posture of the head 10 through the second posture sensor 07.

In some embodiments, the purpose of making the head 10 and the detector 20 to meet the preset position relationship may be to make the head 10 and the detector 20 to close to a centered or aligned relationship. Ideally, the head 10 and the detector 20 form a centered or aligned relationship. For forming the centered or aligned relationship, the second distance requirement may be that the distance is as close to 0 as possible, the irradiation surface of the head 10 and the imaging surface of the detector 20 may be as parallel as possible, and the distance between the head 10 and the imaging surface of the detector 20 may be designed according to the actual situation of the user, such as 1 meter.

The embodiments where the base station 01 or a tag 03 is arranged at the movable detector 20 have been described above.

In some embodiments, the radiographic imaging device 100 may further include a movable assistant device 25. During the detector 20 receiving the radiation rays, the detector 20 may be placed in the assistant device.

Since the assistant device 25 is also movable, and the detector 20 can be placed in the assistant device 25, after the detector 20 is placed in the assistant device 25, the overall component is still movable, that is, it can be placed in the location desired by the technician without being limited to a specific location. For example, when the person being examined is sitting in a wheelchair, the technician can place the assistant device 25 (which contains the detector 20) on the backrest of the wheelchair or under the wheelchair to perform a free position radiography. Alternatively, for a person lying on a transfer bed, the technician can place the assistant device 25 (which contains the detector 20) under the person's body to perform a free position radiography.

In some embodiments, the second support element 32 can be used to place the assistant device 25. When the assistant device 25 is placed on the second support element 32, the second support element 32 can bring the movable detector 20 to move. In such embodiments, after the detector 20 is placed in the assistant device 25, the assistant device can also bring the detector 20 to move.

The assistant device 25 will be described below in conjunction with FIG. 15(1) or FIG. 15(2).

In some embodiments, the movable assistant device 25 includes a main body 26, which defines a space in which the movable detector 20 can be placed or housed. The main body 26 has an opening 28. The opening 28 may be arranged on the upper side of the main body 26. The movable detector 20 can be placed into the assistant device 25 from top to bottom through the opening 28. In some embodiments, the front face of the main body 26 is provided with a hollow area 27, and the area of the hollow area 27 is at least not less than the area of the imaging surface area of the detector 20. Therefore, when the detector 20 is placed in the assistant device 25 with its front face facing forward and its back face facing backward, the main body 26 does not block the imaging surface area of the detector 20, such that the imaging surface area of the detector 20 can receive radiation rays through the hollow area 27.

In the embodiments where the radiographic imaging device 100 includes the movable assistant device 25, the controller 90 can calculate the relative position between the first base station 01 and the tag 03 according to the signals transmitted and received by the first base station 01, where the first base station 01 and the tag 03 are arranged such that the relative position between the first base station 01 and the tag 03 can at least represent the spatial position of the movable assistant device 25.

In some embodiments, the controller 90 calculates the spatial position of the movable assistant device 25 according to the relative position between the first base station 01 and the tag 03. When the movable detector 20 is placed in the movable assistant device 25, the spatial position of the movable detector 20 can be obtained through the spatial position of the movable assistant device 25.

In some embodiments, the movable assistant device 25 is provided with the first posture sensor 05 that is configured to measure the posture of the movable assistant device 25. When the movable detector 20 is placed in the movable assistant device 25, the posture of the movable detector 20 may be obtained through the posture of the movable assistant device 25.

The posture of the movable assistant device 25 may refer to the posture of the assistant device 25 in space, such as one or more of its roll angle, pitch angle and yaw angle. There is a detailed explanation of the posture in the description above, which will not be described here again.

The first posture sensor 05 may be arranged, for example, at the forward facing side or backward facing side of the back face of the main body 26. The first posture sensor 05 may also be arranged, for example, at the corner of the assistant device 25.

In some embodiments, the assistant device 25 may further include a communication device 25a, which is configured for communication, including data transmission. For example, the communication device 25a may transmit the data of the first posture sensor 05, for example, to the controller 90 of the radiographic imaging device 100. In some embodiments, the communication device 25a may be a wireless communication device, such as a Wi-Fi communication device.

In the embodiments where the movable assistant device 25 is provided with the tag 03, the first posture sensor 05 may be provided at the movable detector 20 instead of the movable assistant device 25.

With the movable assistant device 25, some additional components such as one or more of the base station 01, the tag 03 and the first posture sensor 05 can be arranged at the assistant device 25. This way, occupying the precious space of the movable detector 20 itself to arrange one or more of the base station 01, the tag 03 and the first posture sensor 05 can be avoided.

In some embodiments, at least one base station 01, such as the first base station 01 and/or the second base station 01, is arranged in the head 10, such as on the beam limiter 13 of the head 10. At least one tag 03 is arranged at the movable assistant device 25, such as on the corner of the movable assistant device 25. In the embodiments where both the first base station 01 and the second base station 01 are arranged in the head 10, the distance between them is greater than the distance threshold, such that they do not interfere with each other. In some embodiment, the movable assistant device 25 may be provided with one tag 03, two tags 03 or at least three tags 03.

Therefore, the controller 90 can calculate the spatial position of the assistant device 25 through the base station 01 (for example, the first base station 01) arranged in the head 10 and the tag 03 arranged in the movable assistant device 25. For example, the spatial position of the assistant device 25 can be represented by the spatial position of the tag 03 arranged in the assistant device 25. Alternatively, since the position of the tag 03 arranged in the assistant device 25 with respect to the assistant device 25 is known, the spatial position of any location on the assistant device 25 may be obtained according to the spatial position of the tag 03 arranged in the assistant device 25. For example, such location may be the center of the imaging surface of the detector 20 placed in the assistant device 25. The spatial position of the center of the imaging surface of the detector 20 may be used to represent the spatial position of the assistant device 25.

In some embodiments, the controller 90 calculates the spatial position of the movable assistant device 25 according to the relative position between the base station 01, such as the first base station 01, and at least one tag 03.

In some embodiments, at least one base station 01, such as the first base station 01 and/or the second base station 01, may be arranged in a specific location in the space, such as on the ceiling or in a column 92, etc. At least one tag 03 is arranged in the movable assistant device 25, for example, at the corner of the movable assistant device 25. In the embodiments where the first base station 01 and the second base station 01 are respectively arranged in the specific locations in the space, the distance between the base stations is greater than the distance threshold, such that they do not interfere with each other.

Therefore, the controller 90 can calculate the spatial position of the assistant device 25 through the base station 01 (for example, the first base station 01) arranged in the specific location in space and the tag 03 arranged in the movable assistant device 25. Similarly, the spatial position of the assistant device 25 can be represented by the spatial position of the tag 03 arranged in the assistant device 25. Alternatively, since the position of the tag 03 arranged in the assistant device 25 with respect to the assistant device 25 is known, the spatial position of any location on the assistant device 25 may be obtained according to the spatial position of the tag 03 arranged in the assistant device 25. For example, such location may be the center of the imaging surface of the detector 20 placed in the assistant device 25. The spatial position of the center of the imaging surface of the detector 20 may be used to represent the spatial position of the assistant device 25.

In some embodiments, the controller 90 calculates the spatial position of the movable assistant device 25 according to the relative position between one base station 01, such as the first base station 01, and at least one tag 03.

In some embodiments, at least one tag 03 is arranged at the head 10. Similarly, since the controller 90 can obtain the spatial position of the base station 01 (for example, the first base station 01) arranged in a specific location in space, the spatial position of the tag 03 arranged in the head 10 can be calculated according to the relative position between the base station 01 (for example, the first base station 01) and the tag 03 arranged at the head 10 and the spatial position of the base station 01. Therefore, the spatial position of the head 10 can be obtained. For example, the spatial position of the head 10 may be represented by the spatial position of the tag 03 arranged at the head 10. Alternatively, the spatial position of the transmission point of the head 10 may be obtained according to the spatial position of the tag 03 arranged at the head 10. The spatial position of the transmission point of the head 10 may represent the spatial position of the head 10.

In some embodiments, the head 10 is arranged with multiple tags 03. The spatial position of the head 10 may be represented by the spatial position of any one of the tags 03. It is also possible that the spatial position of the transmission point of the head 10 is calculated according to the spatial position of any one of the tags 03 to represent the spatial position of the head 10. The spatial position of the head 10 may also be calculated according to the spatial positions of these tags 03. For example, the spatial position of the transmission point of the head 10 may be calculated to represent the spatial position of the head 10. These calculations can be completed by the controller 90. The posture of the head 10 may also be obtained according to the multiple tags 03 arranged in the head 10, which has been described above and will not be repeated here.

In some embodiments, the head 10 is provided with the second posture sensor 07 configured to measure the posture of the head 10.

In some embodiments, the movable assistant device 25 is provided with multiple tags 03.

The spatial position of the movable assistant device 25 may be represented by the spatial position of any one of the tags 03. Alternatively, the spatial position of the center of the imaging surface of the detector 20 placed in the movable assistant device 25 may be calculated according to the spatial position of any one of the tags 03 to represent the spatial position of the movable assistant device 25. The spatial position of the center of the imaging surface of the detector 20 placed in the movable assistant device 25 may also be calculated according to the spatial positions of these tags 03 to represent the spatial position of the movable assistant device 25. These calculations can be completed by the controller 90.

The posture of the movable assistant device 25 can be obtained through the multiple tags 03 arranged at the movable assistant device 25. In the case where the detector 20 is placed in the assistant device 25, the posture of the assistant device 25 can be considered as the posture of the detector 20.

In some embodiments, the movable assistant device 25 is arranged with multiple tags 03, and the controller 90 calculates the posture of the movable assistant device 25 according to the spatial positions of the multiple tags 03.

In some embodiments, the movable assistant device 25 is provided with at least three tags 03. In some embodiments, the three tags 03 are arranged at three sites of the movable assistant device 25, and the three sites are not on one straight line. For example, the three sites may be located at the three corners of the movable assistant device 25. FIG. 15(1) and FIG. 15(2) show two examples, in which three tags 03 are arranged.

In some embodiments, the controller 90 calculates the spatial position of the movable assistant device 25 according to the relative position between the base station 01, such as the first base station 01, and at least one tag 03.

In some embodiments, the controller 90 calculates the posture of the movable assistant device 25 according to the relative positions between the base station 01, such as the first base station 01, and at least three tags 03.

In some embodiments, the assistant device 25 is provided with the first posture sensor 05 configured to measure the posture of the movable assistant device 25. Therefore, the controller 90 may obtain the posture of the movable assistant device 25 through the first posture sensor 05. When the detector 20 is placed in the assistant device 25, the posture of the assistance device 25 may be regarded as the posture of the detector 20.

It can be seen that the spatial positions and postures of the head 10 and the movable assistant device 25 can be obtained through various ways. Furthermore, calibration may be performed according to the spatial positions and postures of the head 10 and the movable assistant device 25 obtained through different ways.

In some embodiments, the first base station 01 is arranged in the head 10. The movable assistant device 25 is arranged with one or more tags 03, such as three or more tags 03. The movable assistant device 25 is arranged with the first posture sensor 05.

The controller 90 can calculate the spatial position and/or posture of the movable assistant device 25 according to the relative position between the first base station 01 and the tag 03. In the embodiments where the controller 90 calculates the posture of the movable assistant device 25 according to the relative position between the first base station 01 and the tag 03, the controller 90 may also calibrate the posture of the assistant device 25 obtained through the first posture sensor 05 according to the calculated posture.

In the embodiments where the second base station 01 is arranged at the head 10 or at a specific location in space, the controller 90 can calculate the spatial position and/or posture of the movable assistant device 25 according to the relative position between the second base station 01 and the tag 03 arranged at the assistant device 25. Therefore, the controller 90 can calibrate the spatial position of the movable assistant device 25 calculated through the first base station 01 using the spatial position of the movable assistant device 25 calculated through the second base station 01, and the controller 90 can calibrate the posture of the movable assistant device 25 obtained through the first posture sensor 05 using the posture of the assistant device 25 calculated through the second base station 01.

In the embodiments where the second base station 01 is arranged at a specific location in space, the head 10 may be provided with one or more tags 03. The controller 90 may calculate the spatial position and/or posture of the head 10 according to the relative position between the second base station 01 and the tag 03 arranged at the head 10. Therefore, the controller 90 may calibrate the spatial position of the head 10 obtained by the controller 90 through the driving assembly 35 using the spatial position of the head 10 calculated through the second base station 01, and the controller 90 may calibrate the posture of the head 10 obtained by the controller 90 through the driving assembly 35 using the posture of the head 10 calculated through the second base station 01.

In the embodiments where the second base station 01 is arranged at a specific location in space, the head 10 may be provided with one or more tags 03, and the head 10 may also be provided with the second posture sensor 07. The controller 90 may calculate the spatial position and/or posture of the head 10 according to the relative position between the second base station 01 and the tag 03 arranged at the head 10, and the controller 90 may obtain the posture of the head 10 through the second posture sensor 07. Therefore, the controller 90 may calibrate the spatial position of the head 10 obtained by the controller 90 through the driving assembly using the spatial position of the head 10 calculated through the second base station 01. The controller 90 may also calibrate the posture of the head 10 obtained by the second posture sensor 07 using the posture of the head 10 calculated through the second base station 01. Alternatively, the controller 90 may calibrate the posture of the head 10 obtained by the controller 90 through the driving assembly 35 using the posture of the head 10 obtained by the second posture sensor 07.

In some embodiments, the first base station 01 is arranged at a specific location in space. The movable assistant device 25 is provided with one or more tags 03, such as three or more tags 03. The movable assistant device 25 is provided with a first posture sensor 05.

The controller 90 may calculate the spatial position and/or the posture of the movable assistant device 25 according to the relative position between the first base station 01 and the tag 03. In the embodiments where the controller 90 calculates the posture of the movable assistant device 25 according to the relative position between the first base station 01 and the tag 03, the controller 90 may also calibrate the posture of the assistant device 25 obtained by the first posture sensor 05 according to the calculated posture.

Furthermore, the head 10 may be provided with one or more tags 03. The controller 90 may calculate the spatial position and/or the posture of the head 10 according to the relative position between the first base station 01 and the tag 03 arranged at the head 10. Therefore, the controller 90 may calibrate the spatial position of the head 10 obtained by the controller 90 through the driving assembly 35 using the spatial position of the head 10 calculated through the first base station 01, and the controller 90 may also calibrate the posture of the head 10 obtained by the controller 90 through the driving assembly 35 using the posture of the head 10 calculated through the first base station 01.

In the embodiments where the second base station 01 is arranged at a specific location in space, the controller 90 may calculate the spatial position and/or the posture of the movable assistant device 25 according to the relative position between the second base station 01 and the tag 03 arranged at the assistant device 25. Therefore, the controller 90 may calibrate the spatial position of the movable assistant device 25 calculated through the first base station 01 using the spatial position of the movable assistant device 25 calculated through the second base station 01, and the controller 90 may also calibrate the posture of the movable assistant device 25 obtained by the first posture sensor 05 using the posture of the movable assistant device 25 calculated through the second base station 01.

In the embodiments where the second base station 01 is arranged at a specific location in space and the head 10 is provided with one or more tags 03, the controller 90 can calculate the spatial position and/or the posture of the head 10 according to the relative position between the second base station 01 and the tag 03 arranged at the head 10. Therefore, the controller 90 may calibrate the spatial position of the head 10 obtained by the controller 90 through the driving assembly 35 using the spatial position of the head 10 calculated through the second base station 01. Alternatively, the controller 90 may calibrate the spatial position of the head 10 calculated through the first base station 01 using the spatial position of the head 10 calculated through the second base station 01. The controller 90 may also calibrate the posture of the head 10 obtained by the controller 90 through the driving assembly 35 using the posture of the head 10 calculated through the second base station 01. Alternatively, the controller 90 may calibrate the posture of the head 10 calculated through the first base station 01 using the posture of the head 10 calculated through the second base station 01. Alternatively, the controller 90 may calibrate the posture of the detector 20 obtained by the first posture sensor 05 using the posture of the head 10 calculated through the second base station 01.

The embodiments where the base station 01 is arranged at the head 10 or at a specific location in space have been described above. In other embodiments, the base station 10 may be arranged in the movable assistant device 25, which will be explained in detail below.

In some embodiments, at least one tag 03 is arranged at the head 10, such as on the beam limiter 13 of the head 10. At least one base station 01, such as the first base station 01 and/or the second base station 01, is arranged at the movable assistant device 25, such as on the corners of the movable assistant device 25.

Therefore, the controller 90 can calculate the spatial position of the assistant device 25 through the tag 03 arranged at the head 10 and the base station 01 (for example, the first base station 01) arranged at the movable assistant device 25. For example, the spatial position of the assistant device 25 can be represented by the spatial position of the base station 01 (for example, the first base station 01) arranged at the assistant device 25. Alternatively, since the position of the base station 01 (for example, the first base station 01) arranged at the assistant device 25 with respect to the assistant device 25 is known, the spatial position of any location on the assistant device 25 may be obtained according to the spatial position of the base station 01 (for example, the first base station 01) arranged at the assistant device 25. This location may be the center of the imaging surface of the detector 20 placed in the assistant device 25. The spatial position of the center of the imaging surface of the detector 20 placed in the assistant device 25 can represent the spatial position of the assistant device 25.

In some embodiments, the controller 90 calculates the spatial position of the movable assistant device 25 according to the relative position between the tag 03 and at least one base station 01, such as the first base station 01.

In some embodiments, at least one tag 03 is arranged at a specific location in the space, such as at the ceiling or the column 92, etc. At least one base station 01, such as the first base station 01 and/or the second base station 01, is arranged in the movable assistant device 25, for example, at the corners of the movable assistant device 25.

Therefore, the controller 90 can calculate the spatial position of the assistant device 25 through the tag 03 arranged at a specific location in space and the base station 01 (for example, the first base station 01) arranged in the assistant device 25. Similarly, the spatial position of the assistant device 25 can be represented by the spatial position of the base station 01 (for example, the first base station 01) arranged in the assistant device 25. Alternatively, since the position of the base station 01 (for example, the first base station 01) arranged in the assistant device 25 with respect to the assistant device 25 is known, the spatial position of any location on the assistant device can be obtained according to the spatial position of the base station 01 (for example, the first base station 01) arranged in the assistant device 25. This location may be the center of the imaging surface of the detector 20 placed in the assistant device 25. The spatial position of the center of the imaging surface of the detector 20 placed in the assistant device 25 can represent the spatial position of the assistant device 25.

In some embodiments, the controller 90 calculates the spatial position of the movable assistant device 25 according to the relative position between the tag 03 and at least one base station 01, such as the first base station 01.

In some embodiments, at least one base station 01 (e.g., the first base station 01) is arranged in the head 10. Similarly, since the controller 90 can obtain the spatial position of the tag 03 arranged at a specific location in space, the spatial position of the base station 01 arranged in the head 10 (e.g., the first base station 01) can be calculated according to the relative position between the tag 03 and the base station 01 arranged in the head 10 (e.g., the first base station 01) and the spatial position of the tag 03. Therefore, the spatial position of the head 10 can be obtained. For example, the spatial position of the base station 01 arranged in the head 10 (e.g., the first base station 01) can be used to represent the spatial position of the head 10. Alternatively, the spatial position of the transmission point of the head 10 may be obtained according to the spatial position of the base station 01 arranged in the head 10 (e.g., the first base station 01). The spatial position of the transmission point of the head 10 may be used to represent the spatial position of the head 10.

In some embodiments, the head 10 is arranged with multiple base stations 01 (for example, the first base station 01). The spatial position of the head 10 can be represented by the spatial position of any one of the base stations 01 (for example, the first base station 01). Alternatively, the spatial position of the transmission point of the head 10 can be calculated according to the spatial position of any one of the base stations 01 (for example, the first base station 01) to represent the spatial position of the head 10. The spatial position of the head 10 can also be calculated according to the spatial positions of these base stations 01 (for example, the first base station 01). For example, the spatial position of the transmission point of the head 10 can be calculated according to the spatial positions of these base stations 01 (for example, the first base station 01) to represent the spatial position of the head 10. These calculations can be performed by the controller 90. The posture of the head 10 can also be obtained through the multiple base stations 01 (for example, the first base station 01) arranged in the head 10, which has been described above and will not be repeated here.

In some embodiments, the head 10 is arranged with the second posture sensor 07 configured to measure the posture of the head 10.

In some embodiments, the movable assistant device 25 is arranged with multiple base stations 01 (for example, the first base station 01).

The spatial position of the movable assistant device 25 can be represented by the spatial position of any one of the base stations 01 (for example, the first base station 01). Alternatively, the spatial position of any one of the base stations 01 (for example, the first base station 01) may be used to calculate the spatial position of the center of the imaging surface of the detector 20 placed in the assistant device 25 to represent the spatial position of the assistant device 25. The spatial position of the center of the imaging surface of the detector 20 placed in the assistant device 25 may also be calculated according to the spatial position of these base stations 01 (for example, the first base station 01) to represent the spatial position of the assistant device 25. These calculations can be completed by the controller 90.

The posture of the movable assistant device 25 may be obtained according to the multiple base stations 01 (for example, the first base station 01) arranged in the movable assistant device 25. In the case that the detector 20 is placed in the assistant device 25, the posture of the assistant device 25 can be considered as the posture of the detector 20.

In some embodiments, the movable assistant device 25 is arranged with multiple tags 03, and the controller 90 calculates the posture of the movable assistant device 25 according to the spatial positions of the multiple tags 03.

In some embodiments, the movable assistant device 25 is arranged with at least three base stations 01 (e.g., the first base station 01). In some embodiments, the three base stations 01 (e.g., the first base station 01) are arranged at three sites of the movable assistant device 25, and the three sites are not on one straight line. For example, the three sites may be located at the three corners of the movable assistant device 25.

In some embodiments, the controller 90 calculates the spatial position of the movable assistant device 25 according to the relative position between the tag 03 and at least one base station 01, such as the first base station 01.

In some embodiments, the controller 90 calculates the posture of the movable assistant device 25 according to the relative positions between the tag 03 and at least three base stations 01, such as at least three first base stations 01.

In some embodiments, the assistant device 25 is arranged with the first posture sensor 05 configured to measure the posture of the movable assistant device 25. Therefore, the controller 90 may obtain the posture of the movable assistant device 25 through the first posture sensor 05. When the detector 20 is placed in the assistant device 25, the posture of the assistant device 25 may represent the posture of the detector 20.

How to obtain the spatial position and posture of the assistant device 25 through the base station 01 and the tag 03 has been described above.

In some embodiments, the controller may prompt the user according to the relative position between the base station 01 (such as the first base station 01) and the tag 03 and/or the posture of the detector 20, for example, by generating information for display and/or audio playback. The prompt information may be one or more of the following: the spatial position of the detector 20, the posture of the detector 20, the distance between the detector 20 and the head 10, the distance between the head 10 and the imaging surface of the detector 20, the distance between the head 10 and the first straight line, the angle between the irradiation surface of the head 10 and the imaging surface of the detector 20, the indicator indicating the direction and/or distance in which the user moves the head 10, the indicator indicating the angle or direction to which the user turns the head 10, and so on. With these prompts, the user can control the movement of the head 10 such that the head 10 and the detector 20 placed in the assistant device 25 meet the preset position relationship.

The first straight line mentioned above is a straight line that is vertical to and passes through the center of the imaging surface of the detector 20.

In some embodiments, the preset position relationship between the head 10 and the detector 20 placed in the assistant device 25 may include: the distance between the head 10 and the imaging surface of the detector 20 satisfies a first distance requirement, and/or, the distance between the head 10 and the first straight line satisfies a second distance requirement, where the first straight line is a straight line that is vertical to and passes through the center of the imaging surface of the detector.

In some embodiments, the preset position relationship between the head 10 and the detector 20 placed in the assistant device 25may include: the angle between the irradiation surface of the head 10 and the imaging surface of the detector 20 satisfies an angle requirement.

The preset position relationship will be described in details below.

In some embodiments, the controller 90 may control the head 10 and the detector 20 placed in the assistant device 25 to satisfy the preset position relationship through the driving assembly 35. In some embodiments, the controller 90 may control the driving assembly 35 to drive the head 10 and/or the assistant device 25 to move according to the relative position between the base station 01 (such as the first base station 01) and the tag 03 (such as the tag 03 arranged at the assistant device 25), and control the driving assembly 35 to drive the head 10 and/or the assistant device 25 to rotate according to at least the posture of the assistant device 25 (such as the posture obtained through the first posture sensor 05), to make the head 10 and the detector 20 placed in the assistant device 25 to satisfy the preset position relationship.

In some embodiments, the preset position relationship between the head 10 and the detector 20 placed in the assistant device 25 may include: the distance between the head 10 and the imaging surface of the detector 20 satisfies a first distance requirement (e.g., less than a first distance), and/or, the distance between the head 10 and a first straight line satisfies a second distance requirement (e.g., less than a second distance), where the first straight line is a straight line that is vertical to and passes through the center of the imaging surface of the detector.

In some embodiments, the controller 90 calculates the spatial position of the assistant device 25 according to the relative position between the base station 01, such as the first base station 01, and the tag 03 arranged at the assistant device 25. The controller 90 acquires the spatial position of the head 10 and, according to the spatial position of the head 10 and the spatial position of the assistant device 25, controls the driving assembly 35 to drive the head 10 and/or the assistant device 25 to move to make the head 10 and the assistant device 25 to satisfy the preset position relationship, for example, to satisfy the first distance requirement and/or the second distance requirement.

In some embodiments, the controller 90 may obtain the spatial position of the head 10 through the driving assembly 35. In the embodiments where the first base station 01 is arranged at a specific location in space and the head 10 is provided with one or more tags 03, the controller 90 may calculate the spatial position of the head 10 through the relative position between the first base station 01 and the tag 03 arranged at the head 10.

In some embodiments, the preset position relationship between the head 10 and the detector 20 placed in the assistant device 25 may include: the angle between the irradiation surface of the head 10 and the imaging surface of the detector 20 satisfies the angle requirement.

In some embodiments, the controller 90 obtains the posture of the head 10 and, according to the posture of the head 10 and the posture of the assistant device 25 (such as the posture obtained through the first posture sensor 05), controls the driving assembly 35 to drive the head 10 and/or the assistant device 25 to rotate to make the head 10 and the detector 20 placed in the assistant device 25 to meet the angle requirement.

In some embodiments, the controller 90 can obtain the posture of the head 10 through the driving assembly 35. In the embodiments where the first base station 01 is arranged at a specific location in space and the head 10 is provided with one or more tags 03, the controller 90 can calculate the posture of the head 10 through the relative position between the first base station 01 and the tag 03 arranged at the head 10. In the embodiments where the head 10 is provided with the second posture sensor 07, the controller 90 can obtain the posture of the head 10 through the second posture sensor 07.

In some embodiments, referring to FIG. 16 or FIG. 17, in a mobile radiographic imaging device 100, such as a radiographic imaging device 100 having a movable body 91, the base station 01, such as the first base station 01, may be arranged at the head 10. In some embodiments, one or more (for example, at least three) tags 03 may be arranged at the movable detector 20. In some embodiments, in a solution in which the movable assistant device 25 is provided, the tag 03 may be arranged at the assistant device 25 instead of the movable detector 20. For example, one or more (for example, at least three) tags 03 may be arranged at the movable assistant device 25.

What described above is the description of the radiographic imaging device 100.

In an embodiment, a positioning method of the radiographic imaging device 100 is provided. The radiographic imaging device 100 may be the device disclosed in any embodiment of the present disclosure. For example, it may include the head 10, the movable detector 20, the first posture sensor 05, at least one base station 01 (for example, the first base station 01), and at least one tag 03.

Referring to FIG. 18, in some embodiments, the positioning method of the radiographic imaging device 100 may include the following steps.

In step 110, the relative position between the base station 01 (e.g., the first base station 01) and the tag 03 may be calculated according to the signals transmitted and received by the base station 01 (e.g., the first base station 01). The relative position includes the relative distance and the relative angle. In some embodiment, the relative position between the base station 01 (e.g., the first base station 01) and the tag 03 can at least represent the spatial position of the movable detector 20.

The ways for arranging the base station 01 (such as the first base station 01) and the tag 03 may be similar to those in the embodiments above and will not be described again here.

In step 120, the posture of the detector 20 may be obtained through the first posture sensor 05.

The posture of the movable detector 20 may be the detector's posture in space, such as one or more of its roll angle, pitch angle and yaw angle. The detector's posture in space may be defined by one or more of the roll angle, the pitch angle and the yaw angle of the imaging surface of the movable detector 20, or by one or more of the angles between the imaging surface of the movable detector 20 and the X axis, Y axis and Z axis in the XYZ three-dimensional coordinate system. The face of the movable detector 20 that faces the object being examined or the head 10 during use is the front face of the detector 20. The front face of the detector 20 has an area for receiving radiation rays, which can be referred to as the imaging surface (or imaging surface area) of the detector 20.

In step 130, the head 10 and/or the detector 20 may be controlled to move according to the relative position between the base station (such as the first base station 01) and the tag 03 (such as the tag 03 arranged at the detector 20) and be controlled to rotate according to at least the posture of the detector 20 (such as the posture obtained through the first posture sensor 05) to meet the preset position relationship between the head 10 and the detector 20.

In some embodiments, the preset position relationship between the head 10 and the detector 20 may be: the distance between the head 10 and the imaging surface of the detector 20 satisfies the first distance requirement (for example, less than the first distance), and/or the distance between the head 10 and the first straight line satisfies the second distance requirement (for example, less than the second distance), where the first straight line is straight line that is vertical to and passing through the center of the imaging surface of the detector.

In some embodiments, in step 130, the spatial position of the detector 20 may be calculated according to the relative position between the base station 01, such as the first base station 01, and the tag 03 arranged at the detector 20. In step 130, the spatial position of the head 10 may be obtained, and, according to the spatial position of the head 10 and the spatial position of the detector 20, the driving assembly 35 may be controlled to drive the head 10 and/or the detector 20 to move to make the head 10 and the detector 20 to meet the preset position relationship, such as meet the first distance requirement and/or the second distance requirement.

In some embodiments, in step 130, the spatial position of the head 10 may be obtained through the driving assembly 35. In the embodiments where the first base station 01 is arranged at a specific location in space and the head 10 is provided with one or more tags 03, in step 130, the spatial position of the head 10 may be calculated through the relative position between the first base station 01 and the tag 03 arranged at the head 10.

In some embodiments, the preset position relationship between the head 10 and the detector 20 may include: the angle between the irradiation surface of the head 10 and the imaging surface of the detector 20 satisfying the angle requirement (e.g., less than the first angle).

In some embodiments, in step 130, the posture of the head 10 may be obtained, and, according to the posture of the head 10 and the posture of the detector 20 (for example, the posture obtained through the first posture sensor 05), the driving assembly 35 may be controlled to drive the head 10 and/or the detector 20 to rotate to make the head 10 and the detector 20 to meet the angle requirements.

In some embodiments, in step 130, the posture of the head 10 may be obtained through the driving assembly 35. In the embodiments where the first base station 01 is arranged at a specific location in space and the head 10 is provided with one or more tags 03, in step 130, the posture of the head 10 may be calculated through the relative position between the first base station 01 and the tag 03 arranged at the head 10. In the embodiments where the head 10 is provided with the second posture sensor 07, in step 130, the posture of the head 10 may be obtained through the second posture sensor 07.

The present disclosure has been described with reference to various exemplary embodiments. However, those skilled in the art will recognize that changes and modifications may be made to the exemplary embodiments without departing from the scope of the present disclosure. For example, the steps and components for performing the steps may be implemented in different ways (e.g., one or more steps may be deleted, modified, or combined into other steps) depending on the specific application or considering any number of cost functions associated with the operation of the system.

The embodiments above may be implemented in whole or in part by software, hardware, firmware or any combination thereof. In addition, as understood by those skilled in the art, the principles of the present disclosure can be reflected in a computer program product on a computer-readable storage medium, which is pre-installed with computer-readable program codes. Any tangible, non-temporary computer-readable storage medium can be used, including magnetic storage devices (hard disks, floppy disks, etc.), optical storage devices (CD, ROM, DVD, Blu Radiation ray disks, etc.), flash memory and/or the like. The computer program instructions can be loaded onto a general-purpose computer, a special-purpose computer or other programmable data processing device to form a machine, such that the instructions executed on the computer or other programmable data processing device can generate a device that can implement the specified function. The computer program instructions can also be stored in a computer-readable memory, which can instruct the computer or other programmable data processing device to operate in a specific manner, such that the instructions stored in the computer-readable memory can form a manufactured product including an implementation device that implements a specified function. The computer program instructions can also be loaded onto a computer or other programmable data processing device, such that a series of steps are performed in the computer or other programmable device to generate a computer-implemented process, such that the instructions executed in the computer or other programmable device can provide steps for implementing a specified function.

Although the principles of the present disclosure have been shown in various embodiments, many modifications to the structures, arrangements, proportions, elements, materials and components particularly suitable for specific environments and operational requirements can be used without departing from the principles and scope of the present disclosure. The modifications and other changes or amendments above will be included in the scope of the present disclosure.

The present disclosure has been described with reference to various embodiments. However, those skilled in the art will recognize that various modifications and changes can be made without departing from the scope of the present disclosure. Therefore, the description herein will be illustrative rather than restrictive, and all the modifications will be included in the scope of the present disclosure. Similarly, the advantages of the embodiments, other advantages and solutions to the problems have been described above. However, the benefits, advantages, solutions to the problems, and any elements that can produce these benefits, advantages or solutions or can make them clearer, should not be interpreted as critical, essential or necessary. The term "including" and any variants thereof used herein are all non-exclusive inclusions. Therefore, the process, method, article or device including the list of elements not only includes these elements, but also includes other elements that are not explicitly listed or do not belong to the process, method, system, article or device. In addition, the term "coupling" and any other variants used herein refer to physical connections, electrical connections, magnetic connections, optical connections, communication connections, functional connections and/or any other connections.

Those skilled in the art will understand that many changes can be made to the details of the embodiments above without departing from the basic principles of the present disclosure. Therefore, the scope of the present disclosure should be determined by the claims.

## Claims

1. A radiographic imaging device, comprising a head, a controller, a movable detector, a first posture sensor, at least one base station and at least one tag; wherein:
the head is configured to generate radiation rays;
the detector is configured to receive the radiation rays for imaging;
the at least one base stations comprises at least one first base station that is configured to transmit signals to the tag and receive signals returned from the tag, and the controller is configured to calculate a relative position between the first base station and the tag according to the signals transmitted and received by one of the first base station, wherein, the relative position comprises a relative distance and a relative angle, and the first base station and the tag are such arranged that the relative position between the first base station and the tag at least represents a spatial position of the detector; and
the first posture sensor is arranged at the detector and configured to measure a posture of the detector.

2. The radiographic imaging device of claim 1, wherein,
the first base station comprises at least two antennas for transmitting and receiving signals, wherein the at least two antennas comprises a first antenna and a second antenna; and
the controller is configured to calculate a first relative distance between the first antenna and the tag according to signals transmitted and received by the first antenna, calculate a first signal phase difference according to the signals transmitted and received by the first antenna and signals transmitted and received by the second antenna, and calculate the relative position between the first base station and the tag according to the first relative distance and the first signal phase difference, wherein the first signal phase difference is a phase difference between the signals received by the first antenna and the signals received by the second antenna.

3. The radiographic imaging device of claim 2, wherein,
the first base station comprises three antennas for transmitting and receiving signals, wherein the three antennas comprises the first antenna, the second antenna, and a third antenna; and
the controller is configured to calculate a second signal phase difference according to the signals transmitted and received by the first antenna and signals transmitted and received by the third antenna, and calculate the relative position between the base station and the tag according to the first relative distance, the first signal phase difference and the second signal phase difference, wherein the second signal phase difference is a phase difference between the signals received by the first antenna and the signals received by the third antenna.

4. The radiographic imaging device of claim 3, wherein, the three antennas are arranged at three sites of the first base station, and the three sites are located at three vertices of a right triangle.

5. The radiographic imaging device of any one of claims 1 to 4, wherein,
at least one of the first base stations is arranged at a specific location at the head or in space, and at least one of the tags is arranged at the detector;
or,
at least one of the tags is arranged at a specific location at the head or in space, and at least one of the first base stations is arranged at the detector.

6. The radiographic imaging device of any one of claims 1 to 5, wherein, the detector is arranged with one tag, two tags or at least three tags; and preferably, three tags are arranged at three sites of the detector, and the three sites are not on one straight line.

7. The radiographic imaging device of any one of claims 1 to 4, wherein, the detector is arranged with multiple first base stations; and preferably, the detector is arranged with three first base stations; and preferably, the three first base stations are arranged at three sites of the detector and the three sites are not on one straight line.

8. The radiographic imaging device of any one of claims 1 to 7, wherein the controller is further configured to calculate a spatial position of the movable detector according to the relative position between one of the first base stations and at least one of the tags.

9. The radiographic imaging device of claim 8, wherein,
the radiographic imaging device further comprises a driving assembly and a support assembly, wherein, support assembly comprises a first support element and/or a second support element, and the first support element is connected to the head to support the head; the driving assembly drives the head to move through the first support element; the driving assembly further drive the second support element to move, and when the detector is placed on the second support element, the second support element bring the detector to move; and the controller is configured to control the driving assembly to drive the head and/or the detector to move according to the relative position and the posture of the detector to make the head and the detector to meet a preset position relationship;
or,
the radiographic imaging device further comprises a support assembly that comprises a first support element and/or a second support element, wherein, the first support element is connected to the head to support the head; when the detector is placed on the second support element, the second support element bring the detector to move; and the controller is configured to prompt an user according to the relative position and the posture of the detector such that the user can control the head and/or the detector to move to make the head and the detector to meet a preset position relationship.

10. The radiographic imaging device of claim 9, wherein, the preset position relationship between the head and the detector comprises: a distance between the head and an imaging surface of the detector satisfying a first distance requirement, and/or a distance between the head and a first straight line satisfying a second distance requirement, wherein the first straight line is a straight line that is vertical to and passes through a center of the imaging surface of the detector; and the controller being configured to control the driving assembly to drive the head and/or the detector to move according to the relative position comprises:
the controller in configured to calculate a spatial position of the detector according to the relative position; and
the controller is configured to acquire a spatial position of the head and, according to the spatial position of the head and the spatial position of the detector, control the driving assembly to drive the head and/ or the detector to move to make the head and the detector to meet the distance requirement.

11. The radiographic imaging device of claim 9, wherein,
the preset position relationship between the head and the detector comprises: an angle between an irradiation surface of the head and an imaging surface of the detector satisfying an angle requirement; and
the controller is configured to control the driving assembly to drive the head and/or the detector to rotate according to at least the posture of the detector, which comprises: the controller is configured to acquire a posture of the head and, according to the posture of the head and the posture of the detector, control the driving assembly to drive the head and/or the detector to rotate to make the head and the detector to meet the angle requirement.

12. The radiographic imaging device of any one of claims 1 to 11, wherein the controller is further configured to calibrate the posture of the detector according to the relative position; and preferably, the controller being further configured to calibrate the posture of the detector according to the relative position comprises:
the controller is configured to calculate the posture of the detector according to the relative positions between the first base station and three tags; and
the controller is configured to calibrate the posture measure by the first posture sensor according to the posture calculated by the relative positions.

13. The radiographic imaging device of any one of claims 4 to 12, wherein,
the head is arranged with one or more tags, and preferably, the head is arranged with at least three tags; and the controller is configured to calculate a spatial position and/or a posture of the head according to the relative position between the first base station and the tag arranged at the head; or
the head is arranged with a second posture sensor configured to measure a posture of the head.

14. The radiographic imaging device of any one of claims 1 to 13, wherein,
the at least one base station further comprises a second base station, the second base station is configured to transmit signals to the tag and receive signals returned from the tag, and the controller is configured to calculate a relative position between the second base station and the tag according to the signals transmitted and received by the second base station, wherein the relative position comprises a relative distance and a relative angle; and
the controller is further configured to calibrate at least one of a spatial position of the detector, a posture of the detector, a spatial position of the head and a posture of the head according to the relative position between the second base station and the tag.

15. The radiographic imaging device of claim 14, wherein the second base station is arranged at the head or at a specific location in space.

16. The radiographic imaging device of any one of claims 1 to 15, wherein the first base station is a UWB-type base station and the tag is a UWB-type tag.

17. A radiographic imaging device, comprising a head, a controller, a movable detector, a movable assistant device, a first posture sensor, at least one base station and at least one tag; wherein
the head is configured to generate radiation rays;
the detector is configured to receive the radiation rays for imaging;
the assistant device is used to place the detector during the detector receiving the radiation rays, wherein the first posture sensor is arranged at the assistant device and configured to measure a posture of the assistant device;
the at least one base station comprises at least one first base station, wherein the first base station is configured to transmit signals to the tag and receive signals returned from the tag; and
the controller is configured to calculate a relative position between the first base station and the tag according to the signals transmitted and received by one of the first base station, wherein, the first base station and the tag are such arranged that the relative position between the first base station and the tag at least represents a spatial position of the detector.

18. A positioning method for a radiographic imaging device, wherein, the radiographic imaging device comprises a head, a movable detector, a first posture sensor, at least one first base station and at least one tag, the first base station is configured to transmit a signal to the tag and receive a signal returned from the tag; and the method comprises:
calculating a relative position between the first base station and the tag according to the signals transmitted and received by the first base station, wherein, the relative position comprises a relative distance and a relative angle, and the relative position between the first base station and the tag at least represents a spatial position of the detector; and
obtaining a posture of the detector through the first posture sensor;
controlling the head and/or the detector to move according to the relative position and controlling the head and/or the detector to rotate according to at least the posture of the detector to make the head and the detector to meet a preset position relationship.
